# EUROPÄISCHE PATENTSCHRIFT

(11) **EP 1 087 967 B1**
(45) Veröffentlichungstag und Bekanntmachung des Hinweises auf die Patenterteilung: **22.10.2003**
(21) Anmeldenummer: 99927818.7
(22) Anmeldetag: 01.06.1999
(51) Int. Cl.: C07D 413/10, C07D 417/10, C07D 413/14, A01N 43/74

(54) **1,3-OXAZOLIN- UND 1,3-THIAZOLIN-DERIVATE, VERFAHREN ZU IHRER HERSTELLUNG UND IHRE VERWENDUNG ALS SCHÄDLINGSBEKÄMPFUNGSMITTEL**
1,3-OXAZOLINE AND 1,3-THIAZOLINE DERIVATIVES, METHOD FOR PRODUCING THE SAME AND THEIR USE AS PESTICIDES
DERIVES DE 1,3-OXAZOLINE ET DE 1,3-THIAZOLINE, LEUR PROCEDE DE PRODUCTION ET LEUR UTILISATION COMME PARASITICIDES

(30) Priorität: 16.06.1998 DE 19826671
(43) Veröffentlichungstag der Anmeldung: 04.04.2001
(73) Patentinhaber: Bayer CropScience GmbH, 65929 Frankfurt/Main (DE)
(72) Erfinder: SCHNATTERER, Stefan, D-65795 Hattersheim (DE); KERN, Manfred, D-55296 Lörzweiler (DE); SANFT, Ulrich, D-65817 Eppstein (DE); MERTENS,Christina, D-45219 Essen (DE)
(86) Internationale Anmeldenummer: EP9903776
(87) Internationale Veröffentlichungsnummer: WO99065901

(56) Entgegenhaltungen:
- WO-A-95/04726

## Beschreibung

Die Erfindung betrifft Diaryl-1,3-oxazoline und -1,3-thiazoline, Verfahren zu ihrer Herstellung, diese enthaltende Mittel und ihre Verwendung zur Bekämpfung von tierischen Schädlingen, insbesondere Insekten, Spinnmilben, Ektoparasiten und Helminthen.

Bestimmte 1,3-Oxazoline und 1,3-Thiazoline eignen sich auf Grund ihrer biologischen Aktivität zur Bekämpfung von tierischen Schädlingen (siehe z.B. EP-A-0 345 775 und EP-A-0 432 661; WO-A-97/06153 ; WO-A-93/24470 und WO-A-95/04726).

Die Wirkungshöhe und/oder Wirkungsdauer dieser vorbekannten Verbindungen ist jedoch, insbesondere gegen bestimmte Organismen oder bei niedrigen Anwendungskonzentrationen, nicht in allen Anwendungsgebieten völlig zufriedenstellend.

Wegen der vielfältigen Anforderungen an moderne Schädlingsbekämpfungsmittel, beispielsweise was Wirkhöhe, Wirkdauer, Wirkspektrum, Anwendungsspektrum, Toxizität, Kombination mit anderen Wirkstoffen, Kombination mit Formulierungsmitteln oder Synthese angeht, und wegen des möglichen Auftretens von Resistenzen kann die Entwicklung solcher Stoffe jedoch nie als abgeschlossen betrachtet werden, und es besteht beständig ein hoher Bedarf an neuen Verbindungen, die zumindest in Teilaspekten Vorteile gegenüber den bekannten bringen.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe sowie weitere nicht explizit genannte Aufgaben, die aus den hierin diskutierten Zusammenhängen ableitbar oder erschließbar sind durch 1,3-Oxazolin und 1,3-Thiazolin-Derivate der Formel (I), 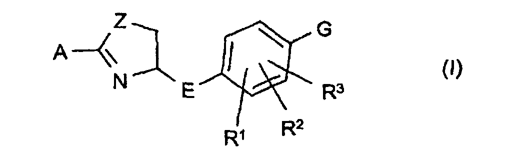 worin die Symbole folgende Bedeutungen haben:
- A: ist Phenyl, Pyridyl, Pyrimidinyl, Pyrazinyl, Pyrazolyl oder Thienyl, wobei jede der genannten Gruppen gegebenenfalls durch einen oder mehrere, vorzugsweise einen, zwei oder drei, Reste X substituiert ist;
- X: ist gleich oder verschieden
a) Halogen, Cyano, Nitro;
b) (C₁-C₄)-Alkyl, (C₂-C₄)-Alkenyl, (C₂-C₄)-Alkinyl, (C₁-C₄)-Alkoxy, (C₁-C₄)-Alkylthio, (C₁-C₄)-Alkylsulfinyl, (C₃-C₈)-Cycloalkyl, (C₄-C₈)-Cycloalkenyl, Phenyl,
   wobei die Reste der Gruppe b gegebenenfalls durch einen oder mehrere, vorzugsweise einen, zwei oder drei, Reste aus der Gruppe Halogen, Cyano, (C₁-C₄)-Alkyl, (C₁-C₄)-Haloalkyl, (C₁-C₄)-Alkoxy und (C₁-C₄)-Haloalkoxy substituiert sind;
- E: ist eine Einfachbindung;
- G: ist ein Rest aus der Gruppe:

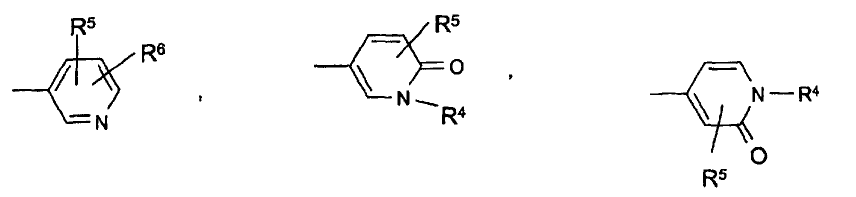

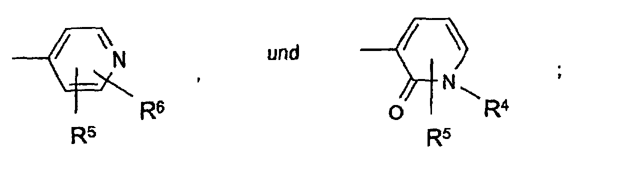

- Z: ist Sauerstoff oder Schwefel;
- R¹, R² und R³: sind gleich oder verschieden Wasserstoff, Halogen, (C₁-C₄)Haloalkyl, (C₁-C₄)Alkyl, (C₁-C₄)Alkoxy, (C₁-C₄)Haloalkoxy oder Cyano;
- R⁴: ist Wasserstoff oder eine Gruppe (C₁-C₈)Alkyl, (C₂-C₈)Alkenyl, (C₂-C₈)Alkinyl, (C₃-C₈)Cycloalkyl, (C₃-C₈)Cycloalkenyl, (C₄-C₁₀)Cycloalkyl-alkyl oder (C₇-C₁₂)Phenylalkyl, wobei jede der genannten Gruppen gegebenenfalls durch einen oder mehrere Substituenten aus der Gruppe Halogen, (C₁-C₄)Alkyl, (C₁-C₃)Haloalkyl, (C₁-C₄)Alkoxy, (C₁-C₃)Haloalkoxy, (C₁-C₃)Alkylthio, (C₃-C₈)Cycloalkyl, (C₇-C₁₂)Phenylalkyoxy, (C₂-C₄)Alkylcarbonyl, Alkoxycarbonyl, (C₂-C₆)Monoalkyl- und (C₃-C₉)Dialkyl-aminocarbonyl, Cyano und Tri(C₁-C₄)alkylsilyl substituiert ist;
- R⁵ und R⁶: sind gleich oder verschieden
a) Wasserstoff, Halogen, Cyano,
b) (C₁-C₈)Alkyl, (C₃-C₈)Cycloalkyl, (C₄-C₈)Cycloalkenyl, (C₁-C₈)Alkylthio, (C₁-C₈)Alkylsulfinyl, (C₁-C₈)Alkylsulfonyl, (C₇-C₁₂)Phenylalkyl, (C₇-C₁₂)Phenylalkoxy, (C₄-C₇)Oxacycloalkyl, (C₄-C₇)Oxacycloalkenyl, wobei jede der Gruppen b gegebenenfalls durch einen oder mehrere, vorzugsweise einen bis drei, Reste aus der Gruppe Halogen, (C₁-C₄)Alkyl, (C₁-C₃)Haloalkyl, (C₁-C₃)Alkoxy, (C₁-C₃)Haloalkoxy, (C₁-C₃)Alkylthio, und Cyano substituiert ist;
c) (C₁-C₈)Alkoxy, wobei jede der Gruppen c gegebenenfalls durch einen oder mehrere, vorzugsweise einen bis drei, Reste aus der Gruppe Halogen, (C₁-C₃)Alkylthio und Cyano substituiert ist; oder
deren reine Isomere (optische und geometrische Isomere) als auch Isomerengemische davon, deren N-Oxide und deren für den Pestizidgebrauch geeigneten Salze.

Überraschenderweise besitzen Verbindungen der Formel (I) eine bessere Akarizid- und Insektizidwirkung hinsichtlich des Wirkungsspektrums und der Wirkstärke als bekannte 1,3-Oxazolin- und 1,3-Thiazolin-Derivate.

Bevorzugt haben die Symbole in der Formel (I) folgende Bedeutungen:
- A: ist vorzugsweise Phenyl oder Pyridyl, besonders bevorzugt Phenyl.
- X: ist bevorzugt
a) Halogen, Cyano, Nitro oder
b) (C₁-C₄)-Alkyl, (C₁-C₄)-Alkenyl, (C₁-C₄)-Alkinyl, (C₁-C₄)-Alkoxy, (C₁-C₄)-Alkylthio, (C₁-C₄)-Alkylsulfinyl, (C₃-C₇)-Cycloalkyl, (C₄-C₈)-Cycloalkenyl,
   wobei die Reste der Gruppe b gegebenenfalls durch einen oder mehrere, vorzugsweise einen, zwei oder drei Reste aus der Gruppe Halogen, Cyano, (C₁-C₄)-Alkoxy und (C₁-C₄)-Haloalkoxy substituiert sind;
- X: ist besonders bevorzugt Halogen, (C₁-C₄)-Alkyl, (C₁-C₃)-Haloalkyl, (C₁-C₄)-Alkoxy oder (C₁-C₃)-Haloalkoxy.
- G: ist bevorzugt 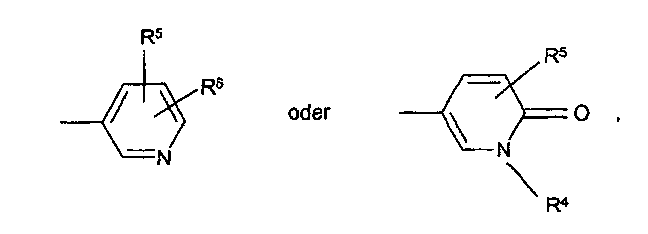 besonders bevorzugt 3-Pyridyl.
- Z: ist bevorzugt Sauerstoff.
- R¹, R², R³: sind bevorzugt H, Halogen, (C₁-C₄)-Haloalkyl, (C₁-C₄)-Alkyl, (C₁-C₄)-Alkoxy, (C₁-C₄)-Haloalkoxy oder Cyano, besonders bevorzugt H, Halogen, (C₁-C₄)-Haloalkyl, (C₁-C₄)-Alkyl, (C₁-C₄)-Alkoxy, (C₁-C₄)-Haloalkoxy,
- R⁴: ist bevorzugt H oder (C₁-C₈)-Alkyl, welches gegebenenfalls durch einen oder mehrere, vorzugsweise 1 bis 3, Reste aus der Gruppe Halogen, (C₁-C₄)-Alkyl, (C₁-C₃)-Haloalkyl, (C₁-C₄)-Alkoxy, (C₁-C₃)-Haloalkoxy, (C₁-C₃)-Alkylthio, (C₃-C₈)-Cycloalkyl, Cyano oder Tri(C₁-C₄)alkylsilyl substituiert ist.

- R⁵, R⁶: sind bevorzugt
a) H, Halogen, Cyano,
b) (C₁-C₈)-Alkyl, (C₁-C₈)-Alkoxy, (C₃-C₈)-Cycloalkyl, (C₄-C₈)-Cycloalkenyl, (C₁-C₈)-Alkylthio, (C₁-C₈)-Alkylsulfinyl, (C₁-C₈)-Alkylsulfonyl, (C₇-C₁₂)- Phenylkalkyl oder (C₇-C₁₂)-Phenylalkoxy,
wobei die Gruppen b durch einen oder mehrere, vorzugsweise 1 bis 3, Reste aus der Gruppe Halogen, (C₁-C₄)-Alkyl, (C₁-C₃)-Haloalkyl und Cyano substituiert sind.

In der obigen Formel ist unter "Halogen" ein Fluor-, Chlor-, Brom- oder lodatom zu verstehen;
unter dem Ausdruck "(C₁-C₄)-Alkyl" ein unverzweigter oder verzweigter Kohlenwasserstoffrest mit 1 bis 4 Kohlenstoffatomen, wie z.B. der Methyl-, Ethyl-, Propyl-, Isopropyl-, 1-Butyl-, 2-Butyl-, 2-Methylpropyl- oder tert.-Butytrest zu verstehen;
unter dem Ausdruck "(C₁-C₈)-Alkyl" die vorgenannten Alkylreste sowie z.B. der Pentyl-, 2-Methylbutyl-, 1,1-Dimethylpropyl-, Hexyl-, Heptyl-, Octyl- oder der 1,1,3,3-Tetramethylbutyl-Rest;
unter dem Ausdruck "(C₁-C₄)-Halogenalkyl" eine unter dem Ausdruck "(C₁-C₄)-Alkyl" genannte Alkylgruppe, in der ein oder mehrere Wasserstoffatome durch die obengenannten Halogenatome, bevorzugt Chlor oder Fluor, ersetzt sind, wie beispielsweise die Trifluormethylgruppe, die 1-Fluorethylgruppe, die 2,2,2-Trifluorethylgruppe, die Chlormethyl-, Fluormethylgruppe, die Difluormethylgruppe oder die 1,1,2,2-Tetrafluorethylgruppe; unter dem Ausdruck "(C₃-C₈)-Cycloalkyl" beispielsweise die Cyclopropyl-, Cyclobutyl- oder Cyclopentylgruppe; sowie der Cyclohexyl-, Cycloheptyl- oder Cyclooctyl-Rest;
unter dem Ausdruck "(C₃-C₈)-Halogencycloalkyl" eine der oben aufgeführten (C₃-C₅)-Cycloalkylreste, in denen eines oder mehrere, im Falle von Fluor gegebenenfalls auch alle Wasserstoffatome, durch Halogen, bevorzugt Fluor oder Chlor, ersetzt sind, wie beispielsweise die 2,2-Difluor- oder 2,2-Dichlorcyclopropan-Gruppe oder der Fluorcyclopentan-Rest;
unter dem Ausdruck "(C₂-C₄)-Alkenyl" z.B. die Vinyl-, Allyl-, 2-Methyl-2-propenyl- oder 2-Butenyl-Gruppe;
unter dem Ausdruck "(C₂-C₄)-Halogenalkenyl" eine (C₂-C₄)-Alkenyl-Gruppe, in der die Wasserstoffatome teilweise oder im Falle von Fluor auch vollständig durch Halogen, bevorzugt Fluor oder Chlor ersetzt sind;
unter dem Ausdruck "(C₂-C₄)-Alkinyl" z.B. die Ethinyl-, Propargyl-, 2-Methyl-2-propinyl oder 2-Butinyl-Gruppe;
unter dem Ausdruck "(C₂-C₈)-Alkinyl" z.B. die vorstehend genannten Reste sowie z.B. die 1-Pentinyl, 2-Pentinyl-, 3-Pentinyl-, 4-Pentinyl- oder die 1-Octinyl-Gruppe; unter dem Ausdruck "(C₂-C₄)-Halogenalkinyl" eine (C₂-C₄)-Alkinylgruppe in der die Wasserstoffatome teilweise, im Falle von Fluor auch vollständig, durch Halogenatome, bevorzugt Fluor oder Chlor, ersetzt sind;
unter dem Ausdruck "(C₁-C₄)-Hydroxyalkyl" z.B. die Hydroxymethyl-, 1-Hydroxyethyl-, 2-Hydroxyethyl-, 1-Hydroxy-1-methyl-ethyl- oder die 1-Hydroxypropyl-Gruppe;
unter dem Ausdruck "(C₁-C₄)-Alkanoyl-(C₁-C₄)alkyl" z.B. eine Acetylmethyl-, Propionylmethyl-, 2-Acetylethyl- oder eine Butyrylmethyl-Gruppe;
unter dem Ausdruck "(C₁-C₄)-Alkanoyl" z.B. die Formyl-, Acetyl-, Propionyl-, 2-Methylpropionyl- oder Butyryl-Gruppe;
unter dem Ausdruck "(C₁-C₈)-Alkanoyl" die vorstehend genannte Reste sowie z.B. die Valeroyl-, Pivaloyl, Hexanoyl-, Heptanoyl- oder Octanoyl-Gruppe;
unter dem Ausdruck "(C₁-C₁₂)-Alkanoyl" z.B. die vorgenannten Reste sowie beispielsweise die Nonanoyl-, Decanoyl- oder die Dodecanoyl-Gruppe;
unter dem Ausdruck "(C₂-C₄)-Halogenalkanoyl" eine (C₁-C₄)-Alkanoyl-Gruppe, in der die Wasserstoffatome teilweise, im Falle von Fluor auch vollständig, durch Halogenatome, bevorzugt Fluor oder Chlor ersetzt sind;
unter dem Ausdruck "(C₂-C₁₂)-Halogenalkanoyl" eine (C₁-C₂₀)-Alkanoyl-Gruppe, in der die Wasserstoffatome teilweise, im Falle von Fluor auch vollständig, durch Halogenatome, bevorzugt Fluor oder Chlor, ersetzt sind;
unter dem Ausdruck "Cyan-(C₁-C₄)-alkyl" eine Cyanalkyl-Gruppe, deren Kohlenwasserstoffrest die unter dem Ausdruck "(C₁-C₄)-Alkyl" angegebenen Bedeutungen hat;
unter den Ausdrücken "(C₁-C₄)-Nitroalkyl" oder "(C₁-C₄)-Thiocyanoalkyl" eine der oben genannten (C₁-C₄)-Alkylgruppen, die mit einer Nitro- oder einer Thiocyano-Gruppe substituiert sind;
unter dem Ausdruck "(C₁-C₄)-Alkoxycarbonyl" z.B. die Methoxycarbonyl-, Ethoxycarbonyl-, Propoxycarbonyl-, Butoxycarbonyl oder tert.-Butoxycarbonyl-Gruppe;
unter dem Ausdruck "(C₁-C₈)-Alkoxycarbonyl" z.B. die vorstehend genannten Reste sowie z.B. die Pentyloxycarbonyl, Hexyloxycarbonyl- oder die Octyloxycarbonyl-Gruppe;
unter dem Ausdruck "(C₁-C₁₂)-Alkoxycarbonyl" die vorstehend genannten Reste sowie z.B. die Nonyloxycarbonyl-, 2-Methyloctyloxycarbonyl-, Decyloxycarbonyl- oder Dodecyloxycarbonyl-Gruppe;
unter dem Ausdruck "(C₁-C₄)-Alkoxycarbonyl-(C₁-C₄)-alkyl" z.B. eine Methoxycarbonylmethyl, Ethoxycarbonylmethyl- oder Methoxycarbonylethyl-Gruppe; unter dem Ausdruck "(C₁-C₄)-Halogenalkoxycarbonyl" eine (C₁-C₄)-Alkoxycarbonyl-Gruppe in der eines oder mehrere, im Falle von Fluor gegebenenfalls auch alle Wasserstoffatome, durch Halogen, bevorzugt Fluor oder Chlor, ersetzt sind;
unter dem Ausdruck "(C₁-C₄)-Alkylthio" eine Alkylthiogruppe, deren Kohlenwasserstoffrest die unter dem Ausdruck "(C₁-C₄)-Alkyl" angegebene Bedeutung hat;
unter dem Ausdruck "(C₁-C₈)-Alkylthio" eine Alkylthiogruppe, deren Alkylrest die unter dem Ausdruck "(C₁-C₈)-Alkyl" angegebene Bedeutung hat;
unter dem Ausdruck "(C₁-C₄)-Halogenalkylthio" eine (C₁-C₄)-Alkylthio-Gruppe, in der ein oder mehrere, im Falle von Fluor gegebenenfalls auch alle Wasserstoffatome des Kohlenwasserstoff-Teils durch Halogen, insbesondere Chlor oder Fluor ersetzt sind;
unter dem Ausdruck "(C₁-C₄)-Alkylsulfinyl" z.B. die Methyl-, Ethyl-, Propyl-, Isopropyl-, Butyl-, Isobutyl-, sek.-Butyl- oder tert.-Butylsulfinyl-Gruppe;
unter dem Ausdruck "(C₁-C₈)-Alkylsulfinyl" eine der vorstehend genannten Alkylsulfinyl-Gruppen sowie z.B. die Pentylsulfinyl-, 2-Methylbutylsulfinyl-, Hexylsuffinyl- oder Octylsulfinyl-Gruppe;
unter dem Ausdruck "(C₁-C₄)-Alkylsulfonyl" z.B. die Methyl-, Ethyl-, Propyl-, Isopropyl-, Butyl-, Isobutyl-, sek.-Butyl- oder tert.-Butylsulfonyl-Gruppe;
unter den Ausdrücken "(C₁-C₄)-Halogenalkylsulfinyl" und "(C₁-C₄)-Halogenalkylsulfonyl" (C₁-C₄)-Alkylsulfinyl- und -sulfonyl-Reste mit den oben angegebenen Bedeutungen, bei denen ein oder mehrere, im Falle von Fluor gegebenenfalls auch alle Wasserstoffatome des Kohlenwasserstoff-Teils durch Halogen, insbesondere Chlor oder Fluor ersetzt sind;
unter den Ausdrücken "Fluormethylsulfinyl" und "Fluormethylsulfonyl" die Mono-, Diund Trifluormethyl-sulfinyl- und -sulfonyl-Gruppe;
unter dem Ausdruck "(C₁-C₄)-Alkoxy" eine Alkoxygruppe, deren Kohlenwasserstoffrest die unter dem Ausdruck "(C₁-C₄)-Alkyl" angegebene Bedeutung hat;
unter dem Ausdruck "(C₁-C₈)-Alkoxy" eine Alkoxy-Gruppe, deren Kohlenwasserstoffrest die unter dem Ausdruck "(C₁-C₈)-Alkyl" angegebene Bedeutung hat;
unter dem Ausdruck "(C₁-C₈)-Alkylsulfonyl" eine der vorstehend genannten Alkylsulfonyl-Gruppen, sowie z.B. die Pentyl-, 2-Methylbutyl-, Hexyl-, Heptyl- oder Octylsulfonyl-Gruppe,
unter dem Ausdruck "(C₁-C₄)-Alkylamino" z.B. die Methylamino-, Ethylamino-, Propylamino-, Isopropylamino-, Butylamino-, Isobutylamino-, sek.-Butylamino- oder die tert.-Butylaminogruppe,
unter dem Ausdruck "(C₁-C₈)-Alkylamino" eine der vorstehend genannten (C₁-C₄)-Alkylamino-Gruppen sowie z.B. die Pentylamino-, Hexylamino-, Heptylamino- oder Octylamino-Gruppe;
unter dem Ausdruck "(C₁-C₄)-Dialkylamino" z.B. die Dimethylamino-, Methyl-ethylamino-, Diethylamino-, Dipropylamino- oder die Dibutylamino-Gruppe, aber auch cyclische Systeme wie z.B. die Pyrrolidino- oder Piperidino-Gruppe,
unter dem Ausdruck "(C₁-C₈)-Dialkylamino" eine der vorstehend genannten (C₁-C₄)-Dialkylamino-Gruppen, sowie z.B. die Dipentyl-, Dihexyl- oder die Dioctylamino-Gruppe;
unter dem Ausdruck "(C₁-C₄)-Halogenalkoxy" eine Halogenalkoxygruppe, deren Halogen-Kohlenwasserstoffrest die unter dem Ausdruck "(C₁-C₄)-Halogenalkyl" angegebene Bedeutung hat;
unter dem Ausdruck "(C₁-C₄)-Alkoxy-(C₁-C₄)-alkyl" beispielsweise eine 1-Methoxyethylgruppe, eine 2-Methoxyethylgruppe, eine 2-Ethoxyethylgruppe, eine Methoxymethyl- oder Ethoxymethylgruppe, eine 3-Methoxypropylgruppe oder eine 4-Butoxybutylgruppe;
unter den Ausdrücken "(C₁-C₄)-Halogenalkoxy-(C₁-C₄)-Alkyl", "(C₁-C₄)-Alkoxy-(C₁-C₄)-halogenalkyl" und "(C₁-C₄)-Halogenalkoxy-(C₁-C₄)-halogenalkyl" (C₁-C₄)-Alkoxy-(C₁-C₄)-alkyl-Reste mit den oben angegebenen Bedeutungen, bei denen eines oder mehrere, im Falle von Fluor gegebenenfalls auch alle Wasserstoffatome der entsprechenden Kohlenwasserstoff-Anteile durch Halogen, bevorzugt Chlor oder Fluor ersetzt sind;
unter dem Ausdruck "(C₁-C₄)-Alkylthio-(C₁-C₄)-alkyl" beispielsweise Methylthiomethyl, Ethylthiomethyl, Propylthiomethyl, 2-Methylthioethyl, 2-Ethylthioethyl oder 3-Methylthiopropyl;
unter dem Ausdruck "Heterocyclyl" ein heteroaromatisches oder heteroaliphatisches Ringsystem, wobei unter "heteroaromatisches Ringsystem" ein Arylrest, worin mindestens eine CH-Gruppe durch N ersetzt ist und/oder mindestens zwei benachbarte CH-Gruppen durch S, NH oder O ersetzt sind, zu verstehen ist, z.B. ein Rest von Thiophen, Furan, Pyrrol, Thiazol, Oxazol, Imidazol, Isothiazol, Isoxazol, Pyrazol, 1.3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Triazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin oder 4H-Chinolizin;
und unter dem Ausdruck "heteroaliphatisches Ringsystem" einen (C₃-C₈)-Cycloalkylrest in dem mindestens eine Kohlenstoff-Einheit durch O, S oder eine Gruppe NR¹¹ ersetzt ist und R¹¹ Wasserstoff, (C₁-C₄)-Alkyl, (C₁-C₄)-Alkoxy oder Aryl bedeutet;
unter dem Ausdruck "Heterocyclyloxy" oder "Heterocyclylthio" einen der oben genannten heterocyclischen Reste die über ein Sauerstoff- oder Schwefelatom verknüpft sind;
unter dem Ausdruck "(C₃-C₈)-Cycloalkoxy" oder "(C₃-C₈)-Cycloalkylthio" eine der oben angeführten (C₃-C₈)-Cycloalkyl-Reste, die über ein Sauerstoff- oder Schwefelatom verknüpft sind;
unter dem Ausdruck "Aryl-(C₁-C₄)-alkanoyl" z.B. die Phenylacetyl-, 3-Phenylpropionyl-, 2-Phenylpropionyl-, 2-Methyl-2-phenyl-propionyl-, 4-Phenylbutyryl- oder die Naphthylacetyl-Gruppe;
unter dem Ausdruck "(C₃-C₆)-Cycloalkyl-(C₁-C₄)-alkanoyl" z.B. die Cyclopropylcarbonyl-, Cyclobutylcarbonyl-, Cyclopentylcarbonyl-, Cyclohexylcarbonyl-, Cyclohexylacetyl- oder die Cyclohexylbutyryl-Gruppe;
unter dem Ausdruck "Heterocyclyl-(C₁-C₄)-alkanoyl" z.B. die Thenoyl-, Furoyl-, Nicotinoyl-, Thienylacetyl- oder die Pyridin-propionyl-Gruppe;
unter dem Ausdruck "(C₃-C₈)-Cycloalkoxycarbonyl" z.B. die Cyclobutyloxycarbonyl-, Cyclopentyloxycarbonyl-, Cyclohexyloxycarbonyl- oder die Cycloheptyloxycarbonyl-Gruppe;
unter dem Ausdruck "(C₃-C₈)-Cycloalkyl-(C₁-C₄)-alkoxycarbonyl" z.B. die Cyclopropylmethoxycarbonyl-, Cyclobutylmethoxycarbonyl-, Cyclopentyloxymethoxycarbonyl, Cyclohexyloxymethoxycarbonyl-, 1-(Cyclohexyl)-ethoxycarbonyl- oder die 2-(Cyclohexyl)-ethoxycarbonyl-Gruppe;

Die oben angegebene Erläuterung gilt entsprechend für Homologe bzw. deren abgeleitete Reste.

Die vorliegende Erfindung betrifft die Verbindungen der Formel (I) in Form der freien Base oder eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren, wie Ameisensäure, Essigsäure, Propionsäure, Malonsäure, Oxalsäure, Fumarsäure, Adipinsäure, Stearinsäure, Ölsäure, Methansulfonsäure, Benzolsulfonsäure oder Toluolsulfonsäure.

Die Verbindungen der Formel (I) weisen zum Teil ein oder mehrere asymmetrische Kohlenstoffatome oder Stereoisomere an Doppelbindungen auf. Es können daher Enantiomere oder Diastereomere auftreten. Die Erfindung umfaßt sowohl die reinen Isomeren als auch deren Gemische. Die Gemische von Diasteromeren können nach gebräuchlichen Methoden, z.B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z.B. durch Salzbildung mit einer chiralen, enantiomerenreinen Säure, Trennung der diastereomeren Salze und Freisetzung der reinen Enantiomeren mittels einer Base.

Ganz besonders bevorzugt sind folgende Gruppen von Verbindungen der Formel (la) bis (le):

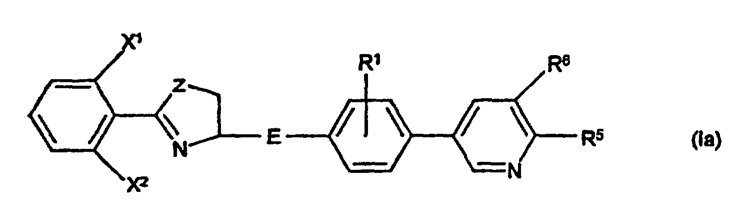

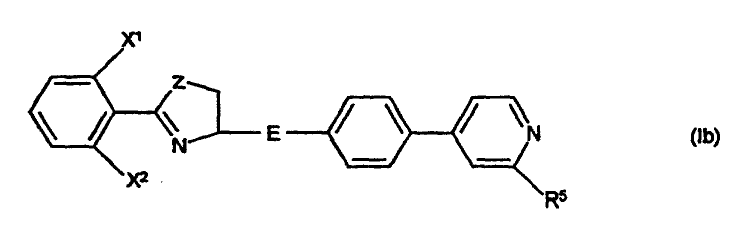

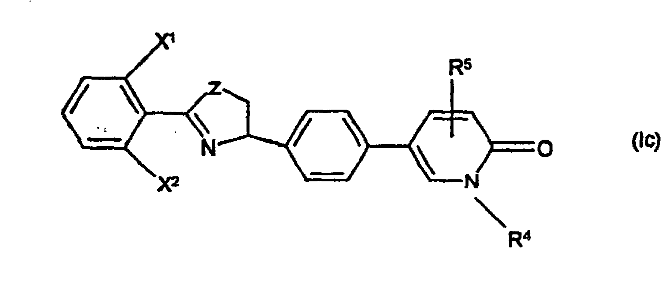

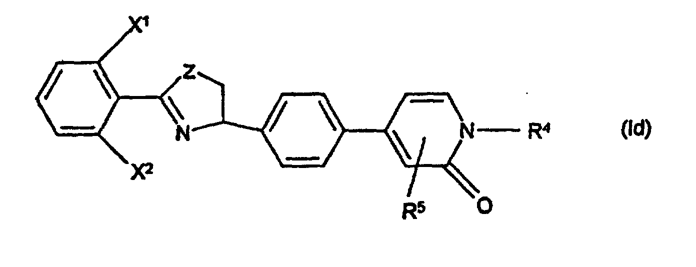

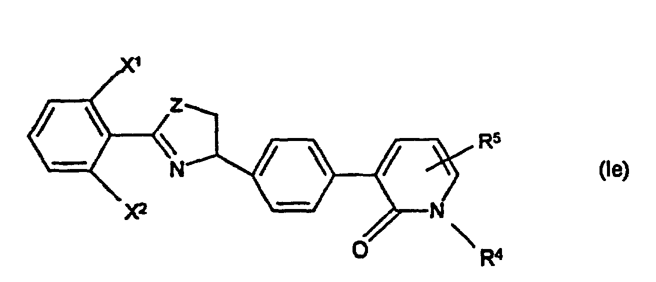

Dabei haben X¹, X², Z, R⁴, R⁵ und R⁶ jeweils die in der Formel (I) angegebenen Bedeutungen (X¹, X² ≙ X).

Insbesondere bevorzugt sind Verbindungen der Formel (la1) bis (la4):

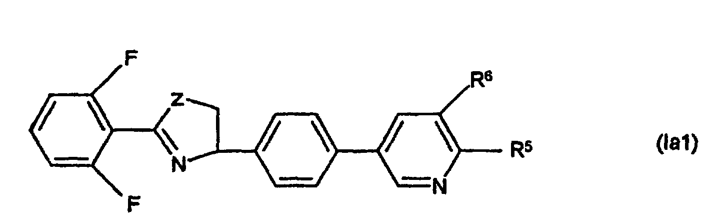

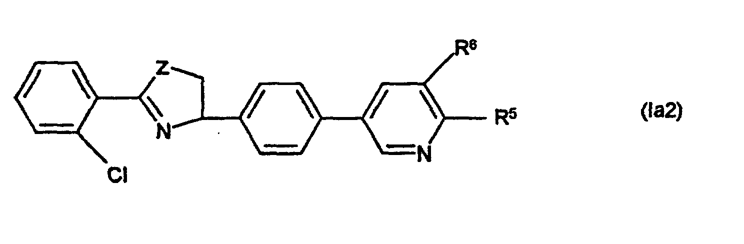

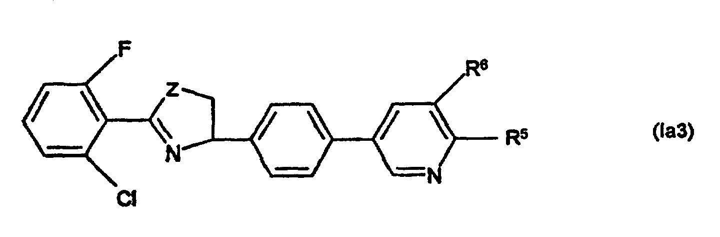

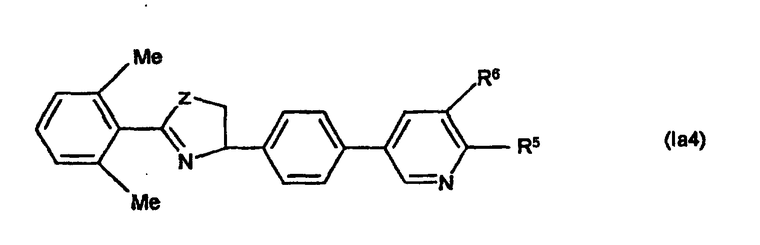

Dabei haben Z, R⁵ und R⁶ jeweils die in der Formel (I) angegebenen Bedeutungen.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben sind.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Die allgemeine Chemie der 1,3-Oxazoline ist beispielsweise beschrieben in Tetrahedron, 1994, 50, 2297-2360 sowie in Nachr. Chem. Tech. Lab. 1996, 44, 744-750.

Gegenstand der Erfindung ist auch ein Verfahren zur Herstellung von Verbindungen der Formel (I) durch die Umsetzung von bekannten 1,3-Oxazolinen und 1,3-Thiazolinen der Formel (II) (siehe z.B. EP-A-0 345 775) (geeignet substituiert mit R¹, R², R³) mit Metalloid-Pyridin-Verbindungen der Formel (III) (geeignet substituiert mit R⁴, R⁵, R⁶). dadurch gekennzeichnet, daß man eine Halogen- und Perfluoralkytsulfonat-Verbindung der Formel (II), 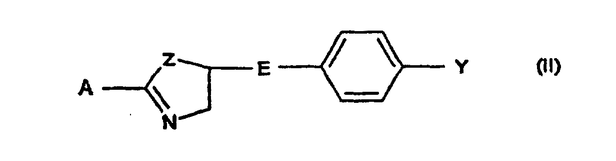 worin
- Y: Cl, Br, I oder Perfluoralkylsulfonat bedeutet und
- A, Z und E: die in der Formel (I) angegebenen Bedeutungen haben,
unter Palladiumkatalyse mit einer metallorganische Verbindung der Formel (III),
G-M (III),
worin
- M: eine B, Sn oder Zn-haltige Abgangsgruppe bedeutet und
- G: die in der Formel (I) angegebenen Bedeutung hat,
umsetzt.

Beispielhaft seien die Methoden A bis H zur Synthese verschiedener Untergruppen von Verbindungen der Formel (I) aufgeführt: 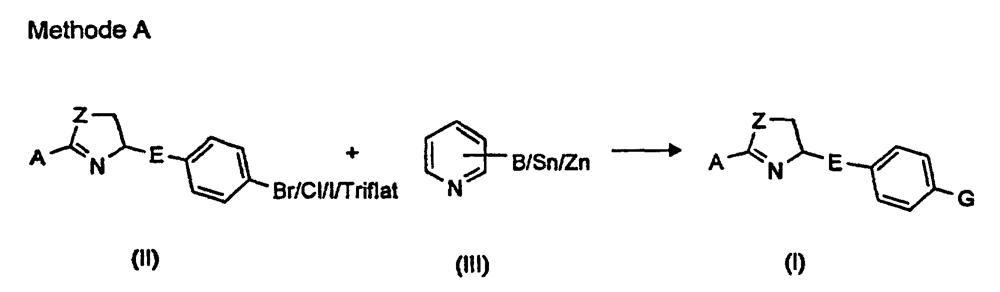 G = 3-Pyridyl, 4-Pyridyl, 2-Pyridon-5-yl, 2-Pyridon-3-yl (vergleiche Formel (I) ).

Die Verknüpfung des Phenylringes mit einem Pyridinring erfolgt beispielsweise durch Palladium-Katalyse unter Anwendung der Suzuki-Kupplung, Stille-Kupplung oder Negishi-Kupplung (siehe z.B. P. Knöchel, Chem. Review 1993, 93, 2117-2188 oder Jiro Tsuji, Palladium Reagents and Catalysts, John Wiley & Sons, 1996).

Die für die Verknüpfung notwendigen Funktionen können auch zwischen (II) und (III) ausgetauscht sein:

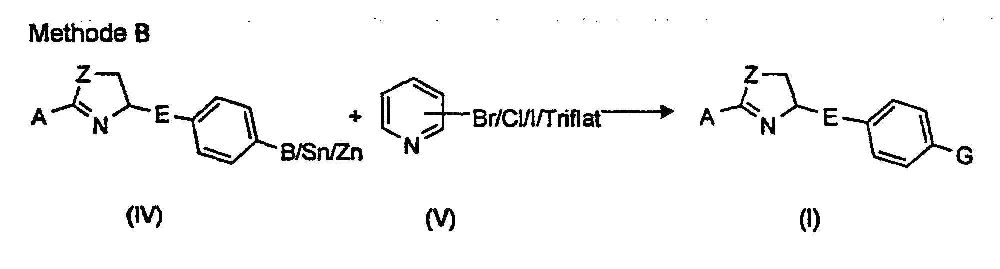

Es ist auch möglich, eine Pyridyl-Phenylstruktureinheit zuerst aufzubauen und diese Vorprodukte in 1,3-Oxazoline und 1,3-Thiazoline der Formel (I) umzuwandeln.

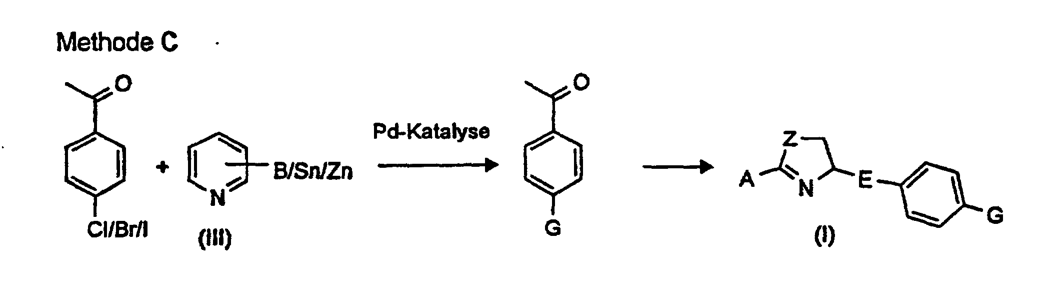

Die Oxazolin-Vorprodukte der Formel (II) werden beispielsweise auf folgenden, zum Teil bekannten Wegen hergestellt (EP-A-0 432 661; G. Helmchen, Tetrahedron 1996, 52, 7547-7583)):

Aktive Carbonsäurederivate werden mit Aminoalkoholen (VI) (geeignet substituiert mit R¹, R², R³) (Synthese K Drauz, J. Org. Chem. 1993, 58, 3568-3571) zu den Amidalkoholen (VII) umgesetzt:

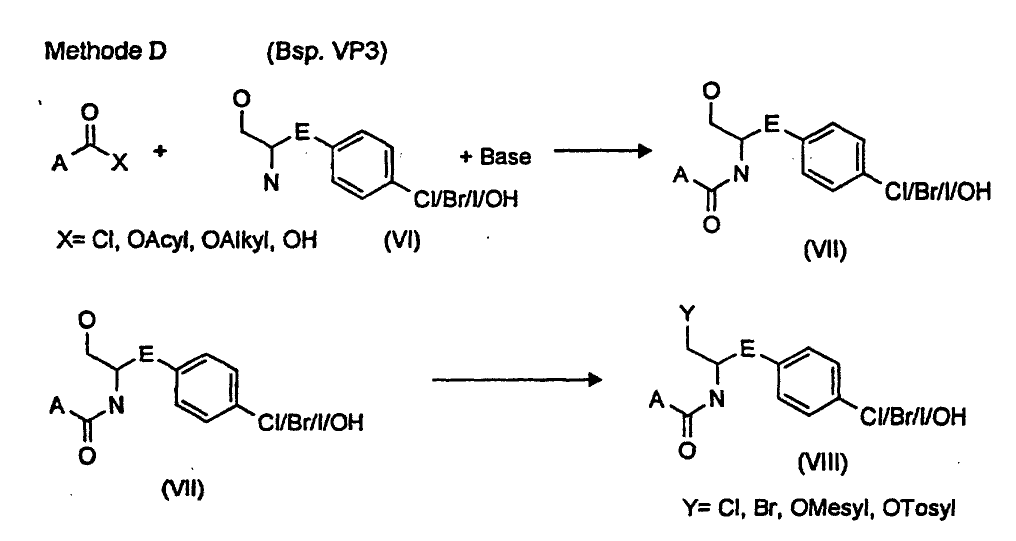

Aus den Amidalkoholen (VII) werden beispielsweise mit Thionylchlorid die Chloride (VIII) (Y=Cl) erhalten, mit Sulfonhalogeniden entstehen die Sulfonate (VIII) (Y= z.B. Mesylat, Tosylat).

Aus (VIII) entstehen durch Baseneinwirkung die Oxazoline (II).

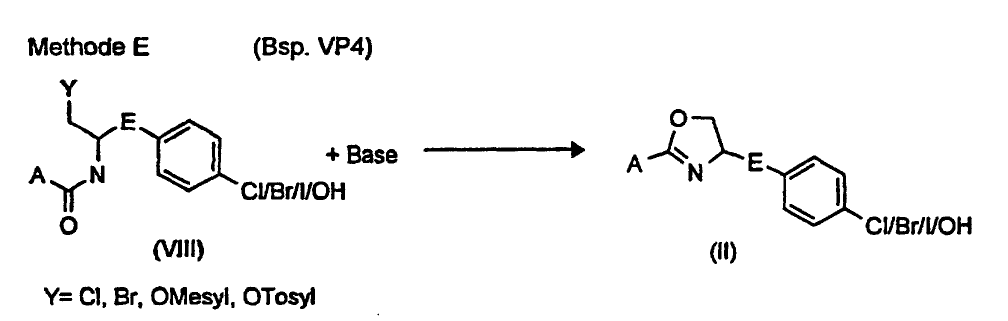

Als Basen geeignet sind beispielsweise basische Salze, wie Alkalihydroxide, Alkalicarbonate, Hydride, Alkoholate und Amine.

Aus den 4-Hydroxyphenylderivaten von (II) können durch Sulfonierung mit beispielsweise Trifluormethansulfochlorid die Triflate erhalten werden.

Die Oxazolin-Varprodukte der Formel (IV) mit einer Zinngruppe sind zugänglich durch eine Stille-Kupplung mit Distannan:

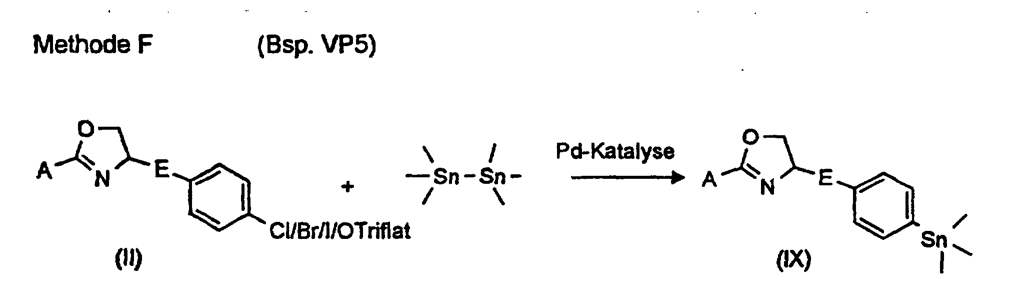

Die Pyridin-Vorprodukte der Formel (III) entstehen z.B. durch Erzeugung von metallierten Pyridinderivaten mit den geeigneten Substituenten R⁴, R⁵, R⁶ gemäß Formel (I) und deren Umsetzungen mit beispielsweise Borsäureestern, Zinnhalogeniden oder Zinkhalogeniden.

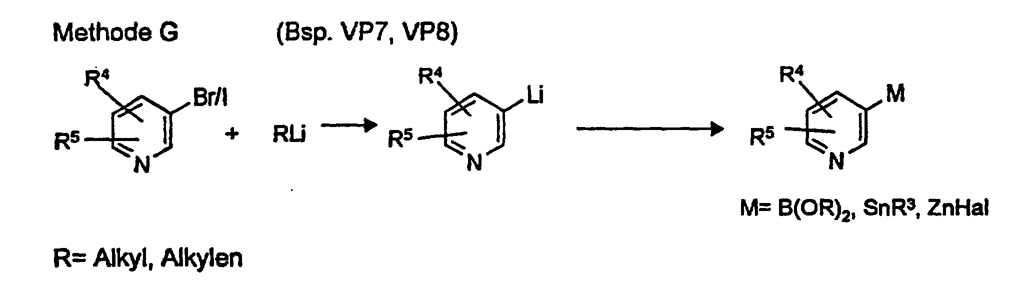

Die Aminoalkohol-Vorprodukte (VI) können durch Hydridreduktion von bekannten 2-Hydroxyacetophenon-oximen hergestellt werden.

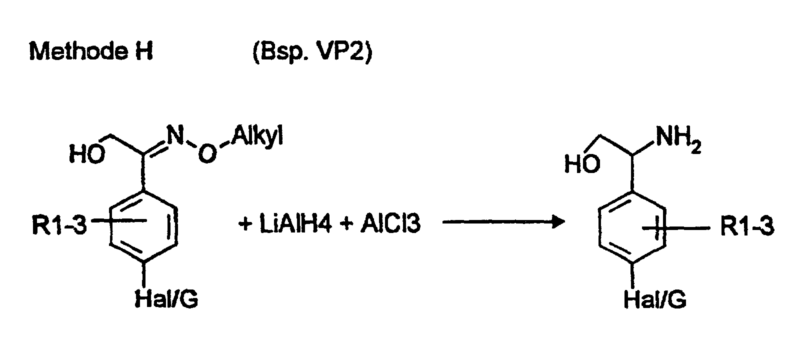

Kollektionen aus Verbindungen der Formel (I), die nach oben genannten Schema synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatorial Chemistry and Molecular Diversity: Automated synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, CM9 8SE, England oder H+P Labortechnik GmbH, Bruckmannring 28, 85764 Oberschleißheim, Deutschland angeboten werden. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben dem hier beschriebenen kann die Herstellung von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z.B. Barry A. Bunin in "The Combinatorial Index", Verlag Academic Press, 1998.

Die Verwendung von Festphasen unterstützten Synthesemethoden erlaubt eine Reihe von literaturbekannten Protokollen, die wiederum manuell oder automatisierten ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die Verbindungen der Formel (I) eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.. Aus der Ordnung der Isopoda z.B. Oniscus aselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung des Isoptera z.B. Reticulitermes spp..
Aus der Ordnung der Anoplura z.B. Phylloera vastatrix, Pemphigus spp., Pediculus humanus corporis, Haematopinus spp., Linognathus spp..
Aus der Ordnung der Mallophaga z.B. Trichodectes pp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex Iectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma Ianigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium comi, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria melloneda, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp..
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopsis, Ceratophyllus spp..
Aus der Ordnung der Arachnida z.B. Scorpio maurus, Latrodectus mactans.
Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola.
Aus der Klasse der Gastropoda z.B. Deroceras spp., Arion spp., Lymnaea spp., Galba spp., Succinea spp., Biomphalaria spp., Bulinus spp., Oncomelania spp.. Aus der Klasse der Bivalva z.B. Dreissena spp..

Zu den pflanzenparasitären Nematoden, die erfindungsgemäß bekämpft werden können, gehören beispielsweise die wurzelparasitären Bodennematoden wie z.B. solche der Gattungen Meloidogyne (Wurzelgallennematoden, wie Meloidogyne incognita, Meloidogyne hapla und Meloidogyne javanica), Heterodera und Globodera (zystenbildende Nematoden, wie Globodera rostochiensis, Globodera pallida, Heterodera trifolii) sowie der Gattungen Radopholus wie Radopholus similis, Pratylenchus wie Pratyglenchus neglectus, Pratylenchus penetrans und Pratylenchus curvitatus;
Tylenchulus wie Tylenchulus semipenetrans, Tylenchorhynchus, wie Tylenchorhynchus dubius und Tylenchorhynchus claytoni, Rotylenchus wie Rotylenchus robustus, Heliocotylenchus wie Haliocotylenchus multicinctus, Belonoaimus wie Belonoaimus longicaudatus, Longidorus wie Longidorus elongatus, Trichodorus wie Trichodorus primitivus und Xiphinema wie Xiphinema index.

Femer lassen sich mit den erfindungsgemäßen Verbindungen die Nematodengattungen Ditylenchus (Stengelparasiten, wie Ditylenchus dipsaci und Ditylenchus destructor), Aphelenchoides (Blattnematoden, wie Aphelenchoides ritzemabosi) und Anguina (Blütennematoden, wie Anguina tritici) bekämpfen.

Die Erfindung betrifft auch Mittel, beispielsweise Pflanzenschutzmittel, vorzugsweise insektizide, akarizide, ixodizide, nematizide, molluskizide oder fungizide, besonders bevorzugt insektizide und akarizide Mittel, die eine oder mehrere Verbindungen der Formel (I) neben geeigneten Formulierungshilfsmitteln enthalten.

Die erfindungsgemäßen Mittel enthalten die Wirkstoffe der Formel (I) im allgemeinen zu 1 bis 95 Gew.-%.

Zur Herstellung der erfindungsgemäßen Mittel gibt man den Wirkstoff und die weiteren Zusätze zusammen und bringt sie in eine geeignete Anwendungsform.

Sie können auf verschiedene Art formuliert werden, je nachdem wie es durch die biologischen und/oder chemisch-physikalischen Parameter vorgegeben ist. Als Formulierungsmöglichkeiten kommen beispielsweise in Frage:
Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in:
Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel, wie Inertmaterialien, Tenside, Lösungsmittel und weitere Zusatzstoffe, sind ebenfalls bekannt und beispielsweise beschrieben in:
Watkins, "Handbook of Insecticide Dust Diluents and Garriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln undloder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmetnan-6.6'disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Cadodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man beispielsweise durch Vermahlen des Wirkstoffes mit fein verteilten festen Stoffen, z.B. Talkum oder natürlichen Tonen, wie Kaolin, Bentonit, Pyrophyllit oder Diatomeenerde. Granulate können entweder durch Verdüsen des Wirkstoffes auf adsorptionsfähiges, granuliertes Inertmaterial oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial hergestellt werden. Auch können geeignete Wirkstoffe in der für die Herstellung von Düngemittelgranulaten üblichen Weise - gewünschtenfalls in Mischung mit Düngemitteln - granuliert werden.

In Spritzpulvem beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,0005 und 10,0 kglha oder mehr Aktivsubstanz, vorzugsweise liegt sie jedoch zwischen 0,001 und 5 kg/ha.

Die erfindungsgemäßen Wirkstoffe können in ihren handelsüblichen Formulierungen sowie in den aus diesen Formulierungen bereiteten Anwendungsformen (siehe die oben angegebenen Mittel) in Mischungen mit anderen Wirkstoffen, wie Insektiziden, Lockstoffen, Sterilantien, Akariziden, Nematiziden, Fungiziden, Molluskiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:

### 1. aus der Gruppe der Phosphorverbindungen

Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Fonofos, Formothion, Fosthiazate (ASC-66824) Heptenophos, Isazophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos, Tetrachlorvinphos, Thiometon, Triazophos, Trichlorphon, Vamidothion;

### 2. aus der Gruppe der Carbamate

Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneamino)-N-methyl-N-(morpholinothio)carbamate (UC 51717), Triazamate;

### 3. aus der Gruppe der Carbonsäureester

Acrinathrin,Allethrin, Alphametrin, 5-Benzyl-3-furylmethyl-(E)-(1R)-cis-2,2-dimethyl-3-(2-oxothiolan-3-ylidenemethyl)cyclopropanecarboxylate, Beta-Cyfluthrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1 RS)-trans-3-(4-tert.butylphenyl)-2,2-dimethylcyclopropanecarboxylate (NCI 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);

### 4. aus der Gruppe der Amidine

Amitraz, Chlordimeform;

### 5. aus der Gruppe der Zinnverbindungen

Cyhexatin, Fenbutatinoxide;

### 6. Sonstige

Abamectin, ABG-9008, Acetamiprid, Anagrapha falcitera, AKD-1022, AKD-3059, ANS-118, Bacillus thuringiensis, Beauveria bassianea, Bensultap, Bifenazate (D-2341), Binapacryl, BJL-932, Bromopropylate, BTG-504, BTG-505. Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden (Thiamethoxam), Diafenthiuron, N-(3,5-Dichlor-4-(1,1,2,3,3,3-hexafluor-1-propyloxy) phenyl)carbamoyl)-2-chlorbenzcarboximidsäureethylester, DDT, Dicofol, Diflubenzuron, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Endosutfan, Ethiprole (Sulfethiprole), Ethofenprox, Etoxazole (YI-5301), Fenazaquin, Fenoxycarb, Fipronil, Fluazuron, Flumite (Flufenzine, SZI-121), 2-Fluoro-5-(4-(4-ethoxyphenyl)-4-methyl-1-pentyl)diphenylether (MTI 800), Granutose- und Kempolyederviren, Fenpyroximate, Fenthiocarb, Flubenzimine, Flucycloxuron, Flufenoxuron, Flufenprox (ICI-A5683), Fluproxyfen, Gamma-HCH, Halofenozide (RH-0345). Halofenprox (MTI-732), Hexaflumuron (DE_473), Hexythiazox, HOI-9004, Hydramethylnon (AC 217300), Lufenuron, Imidacloprid, Indoxacarb (DPX-MP062), Kanemite (AKD-2023), M-020, MTI-446, Ivermectin, M-020. Methoxyfenozide (Intrepid, RH-2485), Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-1111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242, SI-8601. Silafluofen, Silomadine (CG-177), Spinosad, SU-9118, Tebufenozide, Tebufenpyrad (MK-239). Teflubenzuron, Tetradifon, Tetrasul, Thiactoprid, Thiocyclam, Tl-435. Tolfenpyrad (OMI-88), Triazamate (RH-7988), Triflumuron, Verbutin, Vertalec (Mykotal), Yl-5301,

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen. Die Anwendung geschieht in einer den Anwendungsformen angepaßten üblichen Weise.

Gegenstand der Erfindung ist auch ein Verfahren zur Bekämpfung von Schadinsekten, Acarina, Mollusken undloder Nematoden, bei welchem man auf diese oder die von ihnen befallenen Pflanzen, Flächen oder Substrate eine wirksame Menge einer erfindungsgemäßen Verbindung oder eines erfindungsgemäßen Mittels aufbringt

Ebenso Gegenstand der Erfindung ist die Verwendung einer erfindungsgemäßen Verbindung oder eines erfindungsgemäßen Mittels zur Bekämpfung von Schadinsekten, Acarina, Mollusken und/oder Nematoden.

Die erfindungsgemäßen Wirkstoffe eignen sich auch auf dem veterinärmedizinischen Gebiet, vorzugsweise zur Bekämpfung von Endo- und Ektoparasiten, und auf dem Gebiet der Tierhaltung.

Die Anwendung der erfindungsgemäßen Wirkstoffe kann in bekannter Weise geschehen, wie durch orale Anwendung in Form von beispielsweise Tabletten, Kapseln, Tränken oder Granulaten, durch dermale Anwendung in Form beispielsweise des Tauchens (Dippen), Sprühens (Sprayen), Aufgießen (pour-on and spot-on) und des Einpudems, sowie durch parenterale Anwendung in Form beispielsweise der Injektion.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Die erfindungsgemäßen Verbindungen der Formel (I) können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die Verbindungen können bei Rindern z.B. in Dosierungen von 0,01 bis 1 mg/kg Körpergewicht eingesetzt werden.

Überwiegend insektizid, akarizid, molluskizid oder nematodizid wirkende Verbindungen der Formel (I) können in ihren handelsüblichen Formulierungen entweder allein oder in Kombination mit literaturbekannten Fungiziden angewendet werden.

Als literaturbekannte Fungizide, die erfindungsgemäß mit den Verbindungen der Formel (I) kombiniert werden können, sind z.B. folgende Produkte zu nennen: Aldimorph, Andoprim, Anilazine, BAS 480F, BAS 450F, Benalaxyl, Benodanil, Benomyl, Binapacryl, Bitertanol, Bromuconazol, Buthiobate, Captafol, Captan, Carbendazim, Carboxin, CGA 173506, Cyprofuram, Dichlofluanid, Dichlomezin, Diclobutrazol, Diethofencarb, Difenconazol (CGA 169374), Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropimorph, Fentinacetate, Fentinhydroxide, Ferimzone (TF164), Fluazinam, Fluobenzimine,Fluquinconazole, Fluorimide, Flusilazole, Flutolanil, Flutriafol, Folpet, Fosetylaluminium,Fuberidazole, Fulsulfamide (MT-F 651), Furalaxyl, Furconazol, Furmecyclox, Guazatine, Hexaconazole, ICI A5504, Imazalil, Imibenconazole, Iprobenfos, Iprodione, Isoprothiolane, KNF 317, Kupferverbindungen wie Cu-oxychlorid, Oxine-Cu, Cu-oxide, Mancozeb, Maneb, Mepanipyrim (KIF 3535), Metconazol, Mepronil, Metalaxyl, Methasulfocarb, Methfuroxam, MON 24000, Myclobutanil, Nabam, Nitrothalidopropyl, Nuarimol, Ofurace, Oxadixyl, Oxycarboxin, Penconazol, Pencycuron, PP 969, Probenazole, Propineb, Prochloraz, Procymidon, Propamocarb, Propiconazol, Prothiocarb, Pyracarbolid, Pyrazophos, Pyrifenox, Pyroquilon, Rabenzazole, RH7592, Schwefel, Tebuconazole, TF 167, Thiabendazole, Thicyofen, Thiofanatemethyl, Thiram, Totclofos-methyl, Tolylfluanid, Triadimefon, Triadimenol, Tricyclazole, Tridemorph, Triflumizol, Triforine, Validamycin, Vinchlozolin, XRD 563, Zineb, Natriumdodecylsulfonate, Natrium-dodecyl-sulfat, Natrium-C13/C15-alkoholethersulfonat, Natrium-cetostearyl-phosphatester, Dioctyl-natrium-sulfosuccinat, Natrium-isopropyl-naphthalenesulfonat, Natrium-methylenebisnaphthalene-sulfonat, Cetyl-trimethyl-ammoniumchlorid, Salze von langkettigen primären, sekundären oder tertiären Aminen, Alkyl-propyleneamine, Lauryl-pyrimidiniumbromid, ethoxylierte quartemierte Fettamine, Alkyl-dimethyl-benzyl-ammoniumchlorid und 1-Hydroxyethyl-2-alkyl-imidazolin.

Die oben genannten Kombinationspartner stellen bekannte Wirkstoffe dar, die zum großen Teil in C.D.S. Tomlin, S.B. Walker, The Pesticide Manual, 11. Auflage, British Crop Protection Council, Farnham 1997 beschrieben sind.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 95 Gew.-% Wirkstoff, vorzugsweise zwischen 0,0001 und 1 Gew.-% liegen. Die Anwendung geschieht in einer den Anwendungsformen angepaßten üblichen Weise.

Die Anwendung geschieht in einer den Anwendungsformen angepaßten üblichen Weise, beispielsweise dadurch, daß man zur Bekämpfung pathogener Pilze auf diese, oder die von ihnen befallenen Pflanzen, Flächen oder Substrate oder auf Saatgut eine fungizid wirksame Menge eines erfindungsgemäßen Mittels appliziert.

Gegenstand der Erfindung ist auch Saatgut, beschichtet mit einer wirksamen Menge einer erfindungsgemäßen Verbindung oder eines erfindungsgemäßen Mittels.

Die Verbindungen der Formel (I) können auch zur Bekämpfung von Schadorganismen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Emtegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung in wirtschaftlich bedeutenden transgenen Kulturen von Nutz- und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Bei der Anwendung in transgenen Kulturen, insbesondere mit Insektenresistenzen treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Spektrum an Schädlingen, die bekämpft werden können, oder veränderte Aufwandmengen, die für die Applikation eingesetzt werden können.

Gegenstand der Erfindung ist deshalb auch die Verwendung von Verbindungen der Formel (I) zur Bekämpfung von Schadorganismen in transgenen Kulturpflanzen.

Die erfindungsgemäße Verwendung von Verbindungen der Formel (I) bzw. diese enthaltenden Mitteln, beispielsweise als Insektizid, Akarizid, Molluskizid oder Nematizid, schließt auch den Fall ein, bei dem die Verbindung der Formel (I) oder deren Salz erst nach dem Ausbringen, beispielsweise im Insekt, einer Pflanze oder im Boden, aus einer Vorläufersubstanz gebildet wird.

Auf den Inhalt der deutschen Patentanmeldung 198 15 026.1, deren Priorität die vorliegende Anmeldung beansprucht, sowie auf den Inhalt der beiliegenden Zusammenfassung wird hiermit ausdrücklich Bezug genommen; sie gelten durch Zitat als Bestandteil dieser Beschreibung.

Nachfolgende Beispiele dienen zur Erläuterung der Erfindung, ohne daß diese darauf beschränkt wäre.

### A. Herstellungsbeispiele

### Vorprodukt VP1: 4'-Brom-2-hydroxy-acetophenon-O-methyl-oxim

Natriumformiat (244.6 g, 3597 mmol) wurde in Ethanol-Wasser 7:3 (1 L) vorgelegt. Bei ca. 60°C wurde 2,4'-Dibromacetophenon (200 g. 719 mmol) portionsweise zugegeben und danach die Mischung 7 h am Rückfluß erhitzt. Danach wurde Wasser (1 L) zugemischt. Beim Abkühlen kristallisierte das Hydroxyketon und wurde durch Absaugen isoliert. Man erhielt das Hydroxyketon-Intermediat (143.6 g, = 93% Ausbeute) in reinem Zustand. Es wurde in Dioxan-Wasser 9:1 (700 mL) mit O-Methyl-hydroxylamin-hydrochlorid (59.0 g, 706 mmol) und Natriumacetat (57.9 g,706 mmol) vermischt und 3 h am Rückfluß erhitzt. Nach dem Verrühren mit Wasser (1.3 L) und der Extraktion mit Heptan-Ethylacetat 1:1 erhielt man 4'-Brom-2-hydroxy-acetophenon-O-methyl-oxim, 174.3 g, Öl, Gehalt ca. 90%, 2 Isomere.

### Vorprodukt VP2: 2-Amino-2-(4-bromphenyl)ethanol (Aminoalkohol der Formel VI)

4'-Brom-2-hydroxy-acetophenon-O-methyl-oxim (54.8 g, 224 mmol) wurden bei 20°C als THF-Lösung zu einer Mischung aus Lithium-aluminiumhydrid (20 g, 505 mmol) und Aluminiumchlorid (15.3 g, 112 mmol) in THF (350 ml) unter Stickstoff getropft. Nach 4 h wurde hydrolysiert (40 mL Methanol; 200 mL 2N NaOH) und mit Heptan-Ethylacetat extrahiert. Das Rohprodukt (42 g) wurde mit Heptan-Ethylacetat 2:1 umkristallisiert. Man erhielt 2-Amino-2-(4-bromphenyl)ethanol, 29.3 g, farblose Kristalle, Smp. 95°C.

### Vorprodukt VP3: N-[1-(4'-Bromphenyl)-2-hydroxy-ethyl]-2,6-difluorbenzamid (Formel VII)

2-Amino-2-(4-bromphenyl)ethanol (25.0 g, 116 mmol) und Triethylamin (19.5 mL, 139 mmol) wurden in THF (150 mL) vorgelegt und 2,6-Difluorbenzoylchlorid (13.8 mL, 110 mmol) wurde bei 20°C zugetropft. Nach 15 h wurde mit Wasser verrührt und mit Heptan-Ethylacetat extrahiert. Man erhielt das Amid-alkohol-Produkt, 38.6 g, hellgelber Feststoff, Smp. 141°C.

### Vorprodukt VP4: 2-(2,6-Difluorphenyl)-4-(4-bromphenyl)oxazolin (Formel III)

Synthese mit der Thionylchlorid-Alkalihydroxid-Methode Der Amidalkohol VP3 (38.5 g, 108 mmol) wurde in Dichlormethan mit Thionylchlorid (9.7 mL, 130 mmol) vermischt und 5h auf 40°C erhitzt. Die Mischung wurde eingeengt und der Rückstand (=Amidchlorid, 36.5 g) mit 6N NaOH (32.5 mL) in Dioxan (180 mL) am Rückfluß erhitzt (6 h). Die Mischung wurde mit Wasser verrührt und mit Ethylacetat extrahiert. Das Rohprodukt (Öl, 31.8 g) wurde mit Heptan-Ethylacetat 9:1 umkristallisiert. Man erhielt 2-(2,6-Difluorphenyl)-4-(4-bromphenyl)oxazolin, 17.8 g, farblose Kristalle, Smp. 98°C; Kp. 365°C (GC) .

### Synthese mit der Tosylchlorid-Amin-Methode

Der Amidalkohol VP3 (5.0 g, 14.0 mmol) wurde in Dichlormethan (40 mL) mit Triethylamin (5.67 g, 56 mmol) und Tosylchlorid (2.94 g, 15.5 mmol) vermischt und 5 h am Rückfluß erhitzt. Die Mischung wurde eingeengt, der Rückstand mit Wasser verrührt und mit Ethylacetat extrahiert. Das Rohprodukt (4.6 g) wurde mit Ethanol-Wasser 8:2 umkristallisiert. Man erhielt 2-(2,6-Difluorphenyl)-4-(4-bromphenyl)oxazolin, 3.4 g, farblose Kristalle, Smp. 97°C; 1H-NMR (CDCl₃, ppm): 4.24, 4.81, 5.43, Oxazolin; 7.00, 7.43, F₂C₆H₃; 7.23, 7.51, BrC₆H₄ .

### Vorprodukt VP5: 2-(2,6-Difluorphenyl)-4-(4-trimethylstannylphenyl)oxazolin (Formel IX)

VP3 2-(2,6-Difluorphenyl)-4-(4-bromphenyl)oxazolin (16.4 g, 48.8 mmol) wurde in Dioxan unter Stickstoff vorgelegt, Hexamethyl-distannan (20 g, 61.2 mmol), Pd(PPh3)₄ (1.4 g, 1.2 mmol) und Lithiumchlorid (0.38 g, 4.4 mmol) wurden zugegeben und die Mischung 7 h lang am Rückfluß erhitzt. Das Reaktionsgemisch wurde mit Wasser verrührt und mit Heptan-Ethylacetat extrahiert. Das Rohprodukt (19.6 g, Öl) bestand zu etwa 70% aus dem Sn-Produkt. Die Chromatographie-Trennung (Eluent Heptan-Ethylacetat 2:1) lieferte 2-(2,6-Difluorphenyl)-4-(4-trimethylstannylphenyl)oxazolin, 13.4 g, Öl, wurde kristallin, DC Rf 0.59 (VP4 Rf 0.49).

### Vorprodukt VP6: 5-Brom-2-trifluorethoxy-pyridin

In DMF (500 mL) wurde Natriumhydrid (18.6 g, 60%, 464 mmol) unter Stickstoff vorgelegt. Bei 10°C wurde Trifluorethanol (32.2 mL, 443 mmol) zugetropft. Nach 1 h wurde 2,5-Dibrompyridin (100 g, 422 mmol) portionsweise zugegeben und die Mischung in einem Wasserbad 24 h lang bei 20°C gerührt. Danach wurde mit Wasser verrührt und mit Heptan-Ethylacetat 9:1 extrahiert. Die Destillation des Rohproduktes (105 g) lieferte 5-Brom-2-trifluorethoxy-pyridin, 82.1 g (Gehalt 93%), farblose Flüssigkeit, Kp. 97-104°C/18 mbar.
Analog wurden andere Alkoxypyridine und Alkylthiopyridine hergestellt.

### Vorprodukt VP7: 5,5-Dimethyl-2-(2-trifluorethoxypyridin-5-yl)-1,3,2-dioxaborinan

5-Brom-2-trifluorethoxy-pyridin (30.0 g, 117 mmol) wurde in THF-Diethylether 2:1 (250 mL) vorgelegt unter Stickstoff und abgekühlt. Bei ca. -85°C wurde mit der Spritze eine n-Butyllithium-Lösung (2.5 M, 53.9 mL, 135 mmol) zugegeben. Nach 10 min. wurde bei ca. -80°C Borsäure-isopropylester (33.1 mL, 141 mmol) zugetropft. Man ließ auf -10°C kommen, dann wurden Essigsäure (10.1 mL, 176 mmol) und 2,2-Dimethylpropandiol (15.9 g, 152 mmol) zugegeben. Nach 15 h bei 20°C wurde mit Wasser verrührt und mit Heptan-Ethylacetat extrahiert. Das Rohprodukt (31.5 g) wurde mit Heptan-Ethylacetat 95:5 umkristallisiert. Man erhielt 5,5-Dimethyl-2-(2-trifluorethoxypyridin-5-yl)-1,3,2-dioxaborinan, 20.3 g, weißer Feststoff; 1H-NMR (CDCl₃, ppm): 1.02, Me; 3.75, OCH₂; 4.77, PyOCH₂; 6.82, 7.97, 8.50, PyH .
Analog wurden andere 2-Pyridyl-1,3,2-dioxaborinane hergestellt.

### Vorprodukt VPB: 2-Ethoxy-5-trimethylstannyl-pyridin

5-Brom-2-ethoxypyridin (14.9 g, 68 mmol) wurden in THF-Diethylether 2:1 (120 mL) unter Stickstoff vorgelegt. Bei -80°C wurde n-Butyllithium-Hexan-Lösung (2.5 M, 33 mL, 82 mmol) zugegeben. Nach 15 min. wurde Chlortrimethylzinn (16.4 g, 82 mmol), gelöst in THF, zugetropft. Man ließ auf 0°C kommen, verrührte mit Wasser und extrahierte mit Heptan-Ethylacetat. Das Rohprodukt (15.7 g) wurde im Vakuum destilliert. Man erhielt 2-Ethoxy-5-trimethylstannyl-pyridin, 11.2 g, Kp. 66-75°C/1.2 mbar.
Analog wurden andere Stannylpyridine hergestellt.

### Vorprodukt VP9: 5-[4-1-Amino-2-hydroxy-ethyl]-2-trifluorethoxy-pyridin

2-Amino-2-(4-bromphenyl)ethanol VP2 (3.8 g, 17.1 mmol) und 5,5-dimethyl-2-(2-trifluorethoxypyridin-5-yl)-1,3,2-dioxaborinan VP7 (6.55 g, 20.4 mmol) wurden mit Tetrakis(triphenylphosphin)palladium (0.63 g, 3 mol%), Natriumcarbonat (3.7 g, 35.4 mmol) in Toluol-Ethanol-Wasser (8:2:1, 50 mL) 7 h auf Ruckfluss erhitzt. Nach extraktiver Aufarbeitung und Reinigung durch Säulenchromatographie erhielt man 5-[4-(1-Amino-2-hydroxy-ethyl)phenyl]-2-trifluorethoxy-pyridin, 2.2 g, Smp. 129°C.

### Oxazolin-Produkte:

### 2-(2,6-Difluorphenyl)-4-[4-(2-ethoxy-pyridin-5-yl)phenyl]-oxazolin (Bsp. Nr. 2)

2-(2,6-Difluorphenyl)-4-(4-bromphenyl)oxazolin (VP4) (5.00 g, 14.8 mmol) wurde mit 2-Ethoxy-5-trimethylstannyl-pyridin (VP8) (5.06 g, 17.8 mmol), Pd(PPh3)4 (0.68 g, 0.59 mmol) und Lithiumchlorid (0.12 g, 2.9 mmol) in Dioxan (40 mL) vermischt und unter Stickstoff am Rückfluß erhitzt (7 h). Nach Extraktion und Chromatographie erhielt man 2-(2,6-Difluorphenyl)-4-[4-(2-ethoxy-pyridin-5-yl)phenyl]-oxazolin, 3.86 g, Smp. 119°C ; 1H-NMR (CDCl₃, ppm): 1.42, 4.35, OCH₂CH₃; 4.40, 4.84, 5.51, Oxazolin; 6.79, 7.77, 8.36, Pyridin; 7.00, 7.41, 7.54, Phenyl.

### 2-(2,6-Difluorphenyl)-4-[4-(2-trifluorethoxy-pyridin-5-yl)phenyl]-oxazolin (Bsp.Nr. 3)

2-(2,6-Difluorphenyl)-4-(4-bromphenyl)oxazolin (VP4) (2.0 g, 5.9 mmol) wurde mit 2 -Trifluorethoxy-5-trimethylstannyl-pyridin (2.61 g, 7.7 mmol), Tetrakis(triphenylphosphin)palladium (0.22 g, 0.18 mmol) und Lithiumchiorid (0.38 g, 8.9 mmol) in Dioxan (30 mL) vermischt und 9 h am Rückfluß erhitzt. Nach extraktiver Aufarbeitung und Säulenchromatographie erhielt man 2-(2,6-Difluorphenyl)-4-[4-(2-trifluorethoxy-pyridin-5-yl)phenyl]-oxazolin, 1.37 g, farblose Kristalle.
¹⁹F-NMR (CDCl₃): -75.05 (CF₃), -110.45 (ArF).

### 2-(2,6-Difluorphenyl)-4-[4-(2-n-propyloxy-pyridin-5-yl)phenyl]-oxazolin (Bsp.Nr. 4)

2-(2,6-Difluorphenyl)-4-(4-bromphenyl)oxazolin (VP4) (2.0 g, 2.9 mmol) wurde mit 2-nPropyloxy-5-trimethylstannyl-pyridin (4.6 g, 50%, 7.7 mmol), Pd(PPh3)4 (0.35 g, 0.3 mmol) und Lithiumchlorid (50 mg) in Dioxan vermischt und unter Stickstoff am Rüchfluß erhitzt (7 h). Nach Chromatographie und Umkristallisation in Heptan-Ethylacetat 95:5 erhielt man 2-(2,6-Difluorphenyl)-4-[4-(2-n-propyloxy-pyridin-5-yl)phenyl]-oxazolin, 1.21 g, farblose Kristalle;
¹H-NMR (CDCl₃, ppm): 1.05, 1.82, 4.29, OC₃H₇; 4.32, 4.86, 5.51, Oxazolin; 6.80, 7.78, 8.37, Pyridin; 7.02, 7.42, 7.54, Phenyl.

### 2-(2,6-Difluorphenyl)-4-[4-(2-isobutyloxy-pyridin-5-yl)phenyl]-oxazolin (Bsp.Nr. 8)

2-(2,6-Difluorphenyl)-4-(4-bromphenyl)oxazolin (1.00 g, 2.96 mmol) wurde mit 5,5-Dimethyl-2-(2-isobutyloxy-pyridin-5-yl)-1,3,2-dioxaborinan (1.4 g, 80%ig, 4.3 mmol), Pd(PPh3)4 (0.17 g, 0.15 mmol) und Natriumcarbonat (0.62 g, 5.9 mmol) in Toluol-Ethanol-Wasser 8:2:1 (15 mL) vermischt und unter Stickstoff 8 h lang am Rückfluß erhitzL Nach Extraktion und Chromatographie erhielt man 2-(2,6-Difluorpheny))-4-[4-(2-isobutyloxy-pyridin-5-yl)phenyl]-oxazolin, 1.14 g, farbloses Öl.
1H-NMR (CDCl3, ppm): 1.04, 2.11, 4.10, O-Isobutyl; 4.33, 4.84, 5.51, Oxazolin; 7.00, 7.42, 7.55, Phenyl; 6.81, 7.77, 8.37, Pyridin.

### 2-(2,6-Difluorphenyl)-4-[4-(2-trifluorethoxy-pyridin-5-yl)phenyl]-thiazolin (Bsp. Nr. 19)

2-(2,6-Difluorphenyl)-4-(4-bromphenyl)thiazolin (0.90 g, 2.5 mmol, erhalten aus Vorprodukt VP3 und Lawesson's Reagenz in Toluol) wurde mit 2-Trifluorethoxy-5-trimethylstannyl-pyridin (1.21 g, 3.6 mmol), Pd(PPh3)4 (0.15 g) und Lithiumchlorid (0.16 g) in Dioxan (10 mL) vermischt und 8 h auf Rückfluß erhitzt. Nach Extraktion und Chromatographie erhielt man 2-(2,6-Difluorphenyl)-4-[4-(2-trifluorethoxypyridin-5-yl)phenyl]-thiazolin, 0.46 g, beiger Feststoff; MS: M⁺ 450.
¹H-NMR (CDCl₃, ppm): 3.45, 3.94, 5.83, Thiazolin; 4.82, OCH₂CF₃; 6.95, 7.87, 8.36, Pyridin; 7.00, 7.40, 7.53, Phenyl.

### 2-(2,6-Difluorphenyl)-4-[4-(2-n-propyl-pyridin-5-yl)phenyl]-oxazolin (Bsp.Nr. 34)

2-(2,6-Difluorphenyl)-4-(4-bromphenyl)oxazolin (0.8 g, 2.4 mmol) wurde mit Dimethyl-2-(2-n-propyl-pyridin-5-yl)-1,3,2-dioxaborinan (0.72 g, 3.1 mmol), Pd(PPh₃)₄ (0.14 g, 0.12 mmol) und Natriumcarbonat (0.5 g) in Toluol-Ethanol-Wasser 8:2:1 (11 mL) 8 h auf Rückfluß erhitzt. Nach Extraktion und Chromatographie erhält man 2-(2,6-Difluorphenyl)-4-[4-(2-n-propyl-pyridin-5-yl)phenyl]-oxazolin, 0.20 g, heller Feststoff.
¹H-NMR (CDCl₃, ppm): 1.01, 1.80, 2.81, n-Propyl; 4.34, 4.85, 5.53, Oxazolin; 7.00, 7.44, 7.59, Phenyl; 7.21, 7.79, 8.76, Pyridyl.

### 2-(2,8-Difluorphenyl)-4-[4-(2-ethylsulfinyl-pyridin-5-yl)phenyl]-oxazolin (Bsp. Nr. 40)

2-(2,6-Difluorphenyl)-4-[4-(2-ethylthio-pyridin-5-yl)phenyl]-oxazolin (Bsp. Nr. 39; 0.60 g, 1.5 mmol) wurde mit 3-Chlorperbenzoesäure (0.41 g, 1.7 mmol) in Dichlorethan (10 mL) 5 h bei 20°C verrührt. Nach Extraktion und Chromatographie erhielt man 2-(2,6-Difluorphenyl)-4-[4-(2-ethylsulfinyl-pyridin-5-yl)phenyl]-oxazolin, 0.21 g, farbloses Öl.
¹H-NMR (CDCl₃, ppm): 1.24, 2.98, 3.21, SO-CH₂CH₃; 4.33, 4.87, 5.56, Oxazolin; 7.01, 7.48, 7.64, Phenyl; 8.03, 8.11, 8.84, Pyridin .

### 2-(2,6-Difluorphenyl)-4-[4-(2-(N-acetyl-ethylamino)pyridin-5-yl)phenyl]-oxazolin (Bsp.Nr. 51)

2-(2,6-Difluorphenyl)-4-(4-trimethylstannylphenyl)oxazolin (0.84 g, 2.0 mmol) wurde mit 2-(N-Acetyl-ethylamino)-5-iodpyridin (0.93 g, 3.2 mmol), Pd(PPh₃)₄ (0.12 g) und Lithiumchlorid (0.15 g) in Dioxan (10 mL) vermischt und 8 h am Rückfluß erhitzt. Nach chromatographischer Trennung (Eluent Ethylacetat) erhielt man 2-(2,6-Difluorphenyl)-4-[4-(2-(N-acetyl-ethylamino)pyridin-5-yl)phenyl]-oxazolin, 0.31 g, helles Öl.
¹H-NMR (CDCl₃, ppm): 1.19, 3.94, NC₂H₅; 2.08, COCH₃; 4.33, 4.87, 5.54, Oxazolin; 7.00, 7.43, 7.48, 7.61, Phenyl; 7.32, 7.96, 8.75, Pyridin .

### 2-(2,6-Difluorphenyl)-4-[4-(1-n-propyl-2-pyridon-5-yl)phenyl]-oxazolin (Bsp.Nr. 152)

2-(2,6-Difluorphenyl)-4-(4-trimethylstannylphenyl)oxazolin (0.5 g, 1.2 mmol) wurde mit 1-n-Propyl-5-brom-2-pyridon (0.51 g, 2.4 mmol), Pd(PPh₃)₄ (70 mg) und Lithiumchlorid (0.20 g) in Dioxan (10 mL) vermischt und unter Stickstoff 8 h lang am Rückfluß erhitzt. Nach extraktiver Aufarbeitung und Chromatographie (Eluent Ethylacetat) erhielt man 2-(2,6-Difluorphenyl)-4-[4-(1-n-propyl-2-pyridon-5-yl)phenyl]-oxazolin, 0.20 g, Öl.
1H-NMR (CDCI3, ppm): 1.00, 1.81, 3.96 NC3H7; 4.30, 4.83, 5.49, Oxazolin; 6.64, Pyridon; 7.00, 7.4-7.7, Phenyl und Pyridon.

### 2-(2-Chlorphenyl)-4-[4-(2-trifluorethoxy-pyridin-5-yl)phenyl]-oxazolin (Bsp. Nr. 25)

2-(2-Chlorphenyl)-4-(4-bromphenyl)oxazolin (hergestellt analog VP4) (0.80 g, 2.4 mmol) und 5,5-Dimethyl-2-(2-trifluorethoxypyridin-5-yl)-1,3,2-dioxaborinan VP7 (1.03 g, 3.6 mmol) wurden mit Pd(PPh₃)₄ (0.1 g) und Natriumcarbonat (0.5 g) in Toluol-Ethanol-Wasser (8:2:1, 11 mL) am Rückfluss erhitz (8 h). Nach Extraktion und Säulenchromatographie erhielt man 2-(2-Chlorphenyl)-4-[4-(2-trifluorethoxypyridin-5-yl)phenyl]-oxazolin als helles Öl, 0.86 g; ¹H-NMR (CDCl₃): 4.31, 4.80, 5.50, Oxazolin; 4.80 OCH₂CF₃; 6.93, 7.3 - 7.5, 7.84, 8.35, Pyridin und Phenyl.

### 2-(2,6-Difluorphenyl)-4-[2-ethoxy-4-(2-trifluorethoxy-pyridin-5-yl)phenyll-oxazolin (Bsp. Nr. 106)

Bsp. 106 wurde analog Bsp. Nr. 25 hergestellt.
¹H-NMR (CDCl₃): 1.45, 4.15 OEt; 4.45, 4.92, 5.73, Oxazolin, 4.81 OCH₂CF₃; 6.94, 7.84, 8.35, Pyridin; 6.98, 7.12, 7.41, 7.53, Phenyl.

### 2-(2-Fluorphenyl)-4-[4-(2-trifluorethoxy-pyridin-5-yl)phenyl]-oxazolin (Bsp. Nr. 402)

Bsp. Nr. 402 wurde analog Bsp. Nr. 25 hergestellt.
¹H-NMR (CDCl₃): 4.31, 4.82, 5.48, Oxazolin; 4.80, OCH2CF3; 6.92, 7.84, 8.35, Pyridin; 7.20, 7.40, 7.50, 8.00, Phenyl.

### A. Chemische Beispiele (Tabellen 1 - 8)

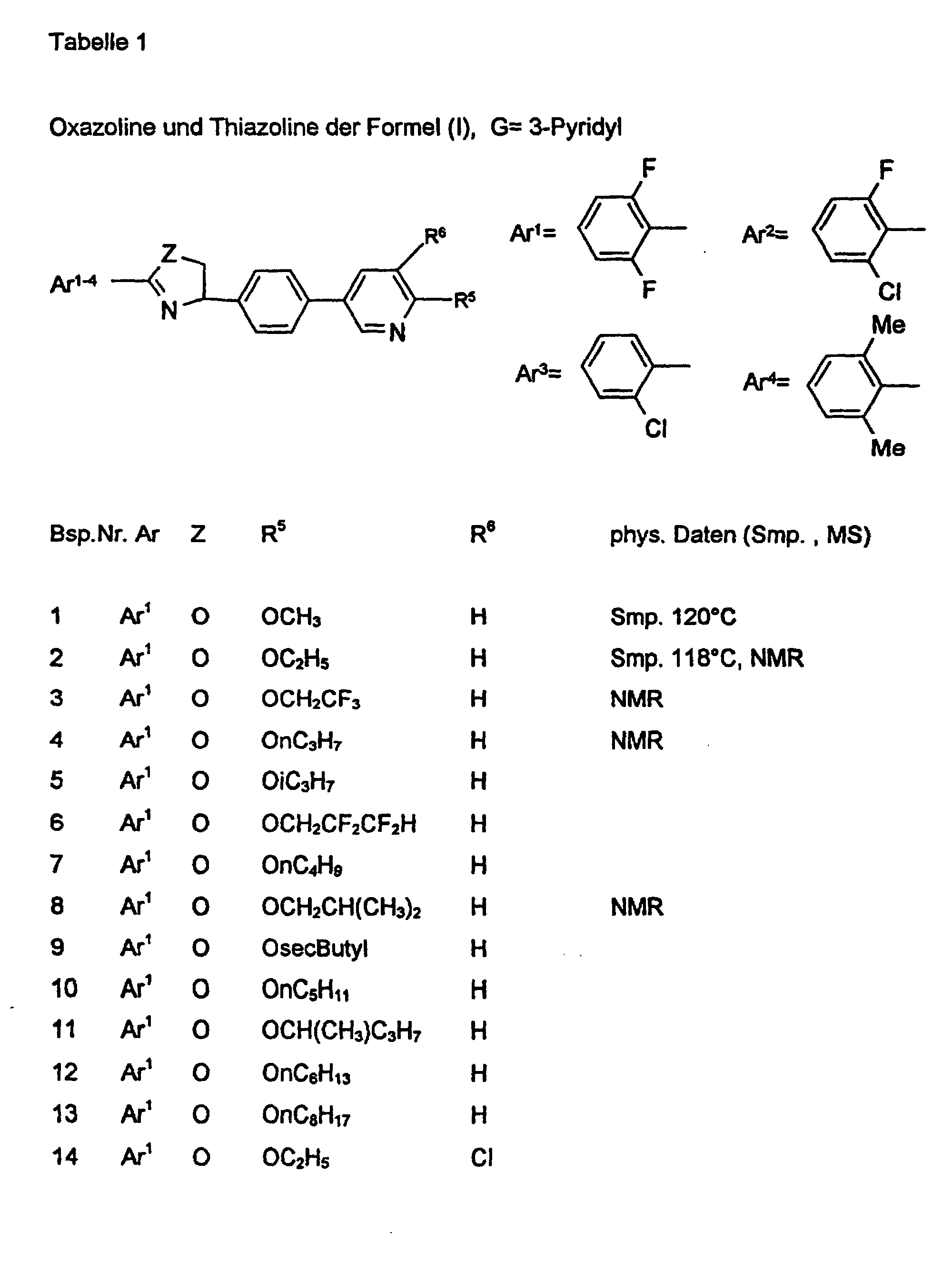

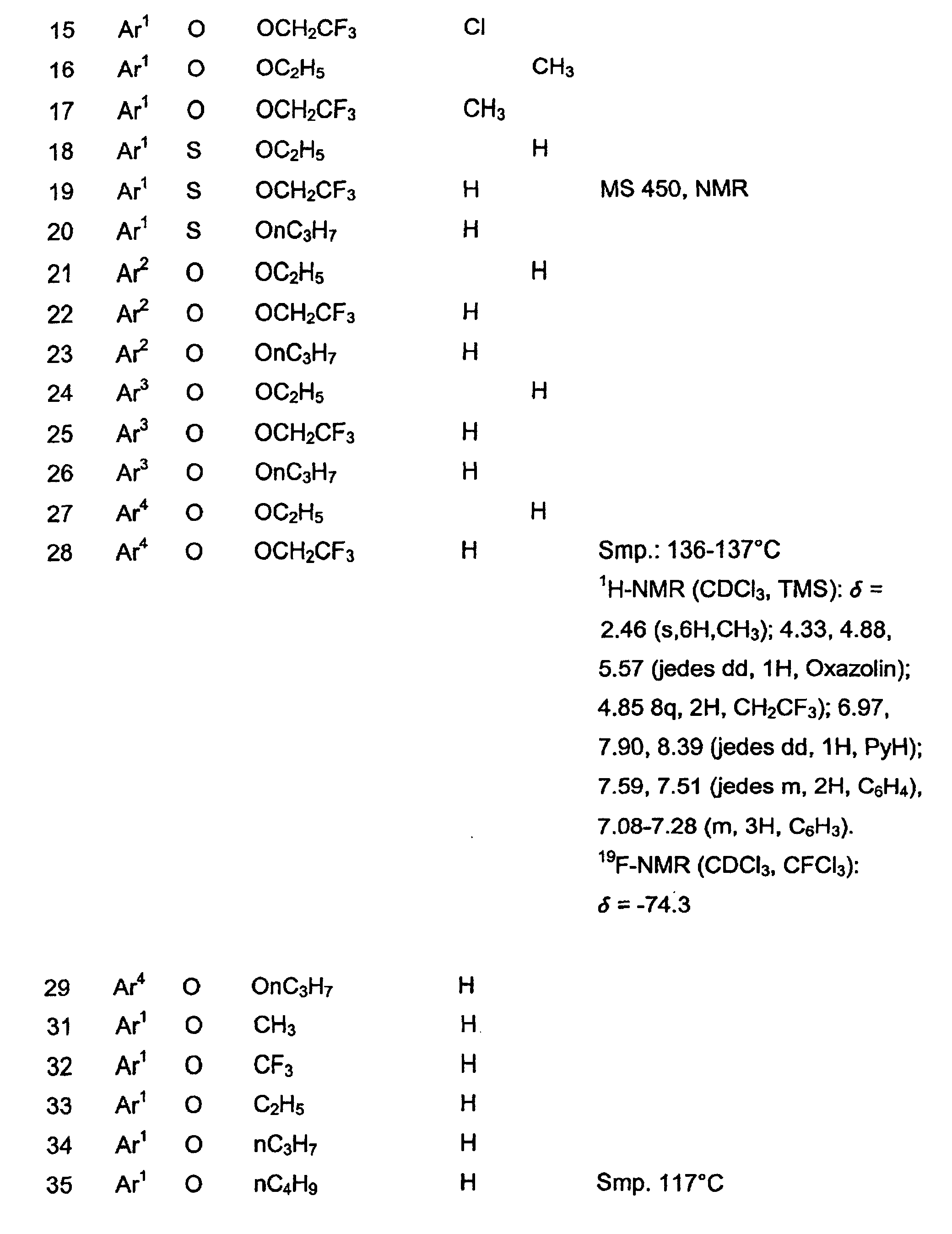

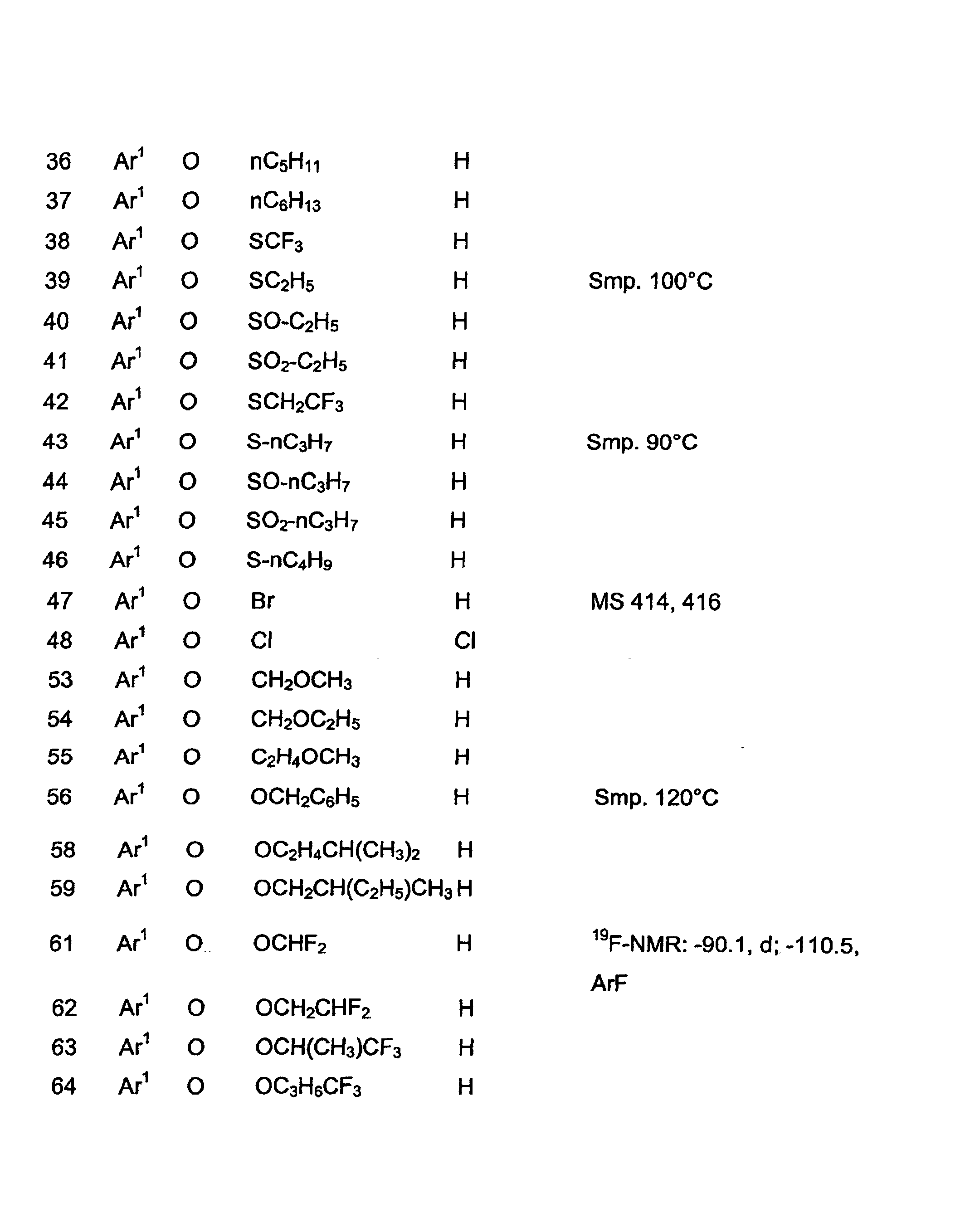

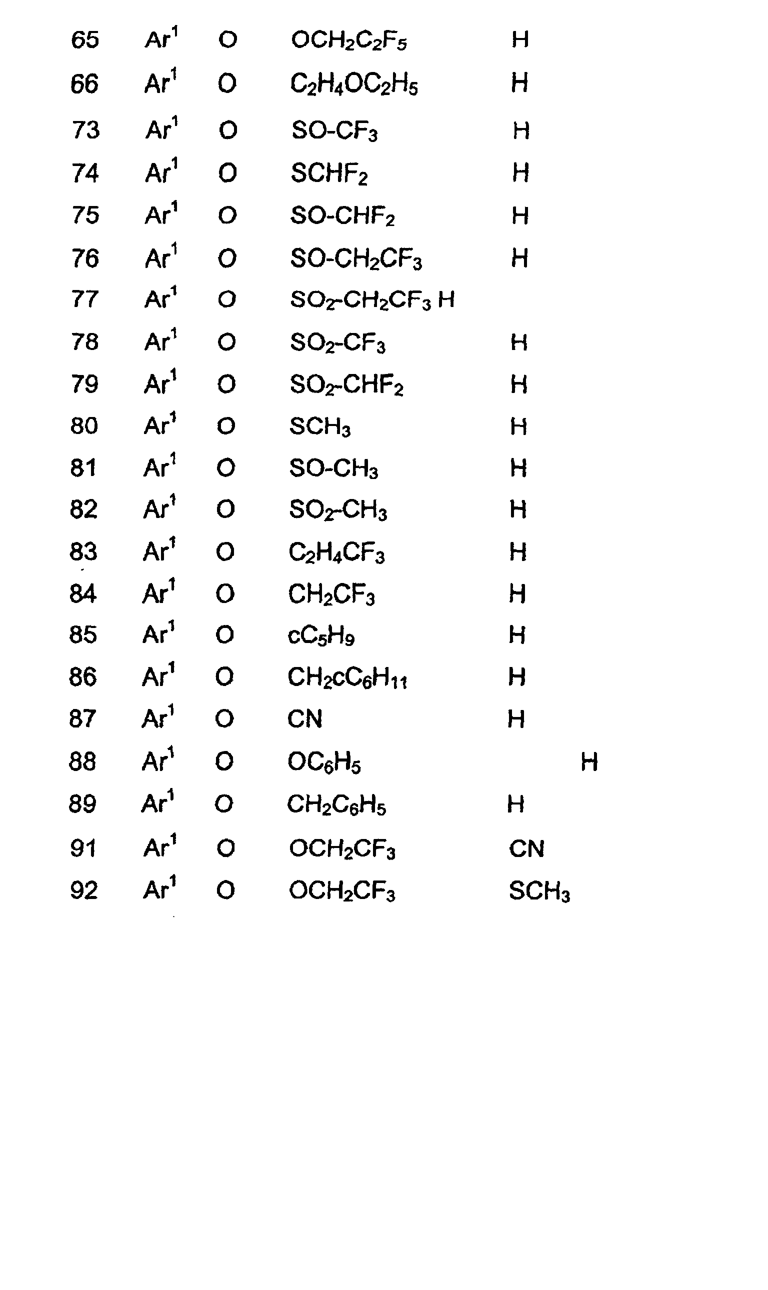

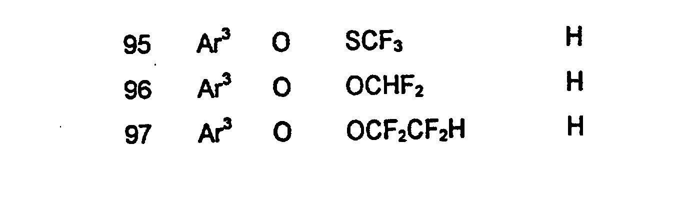

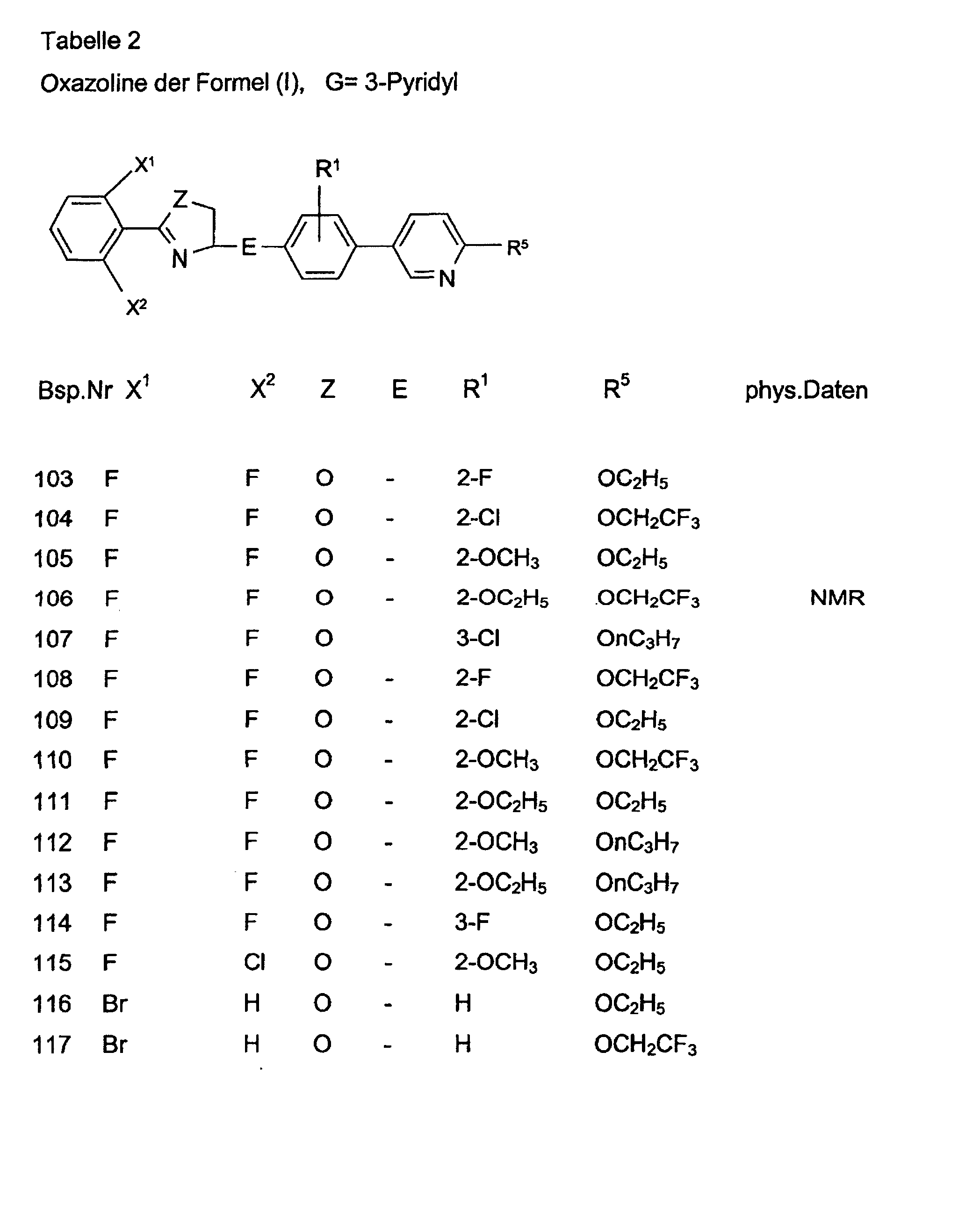

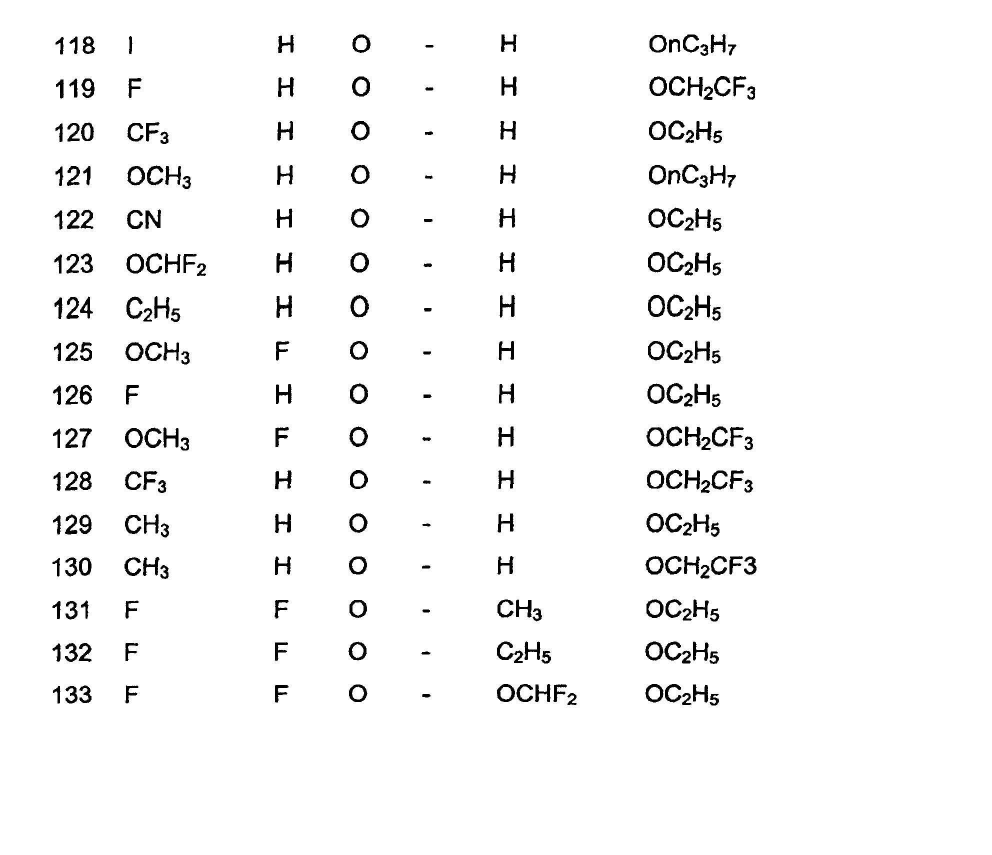

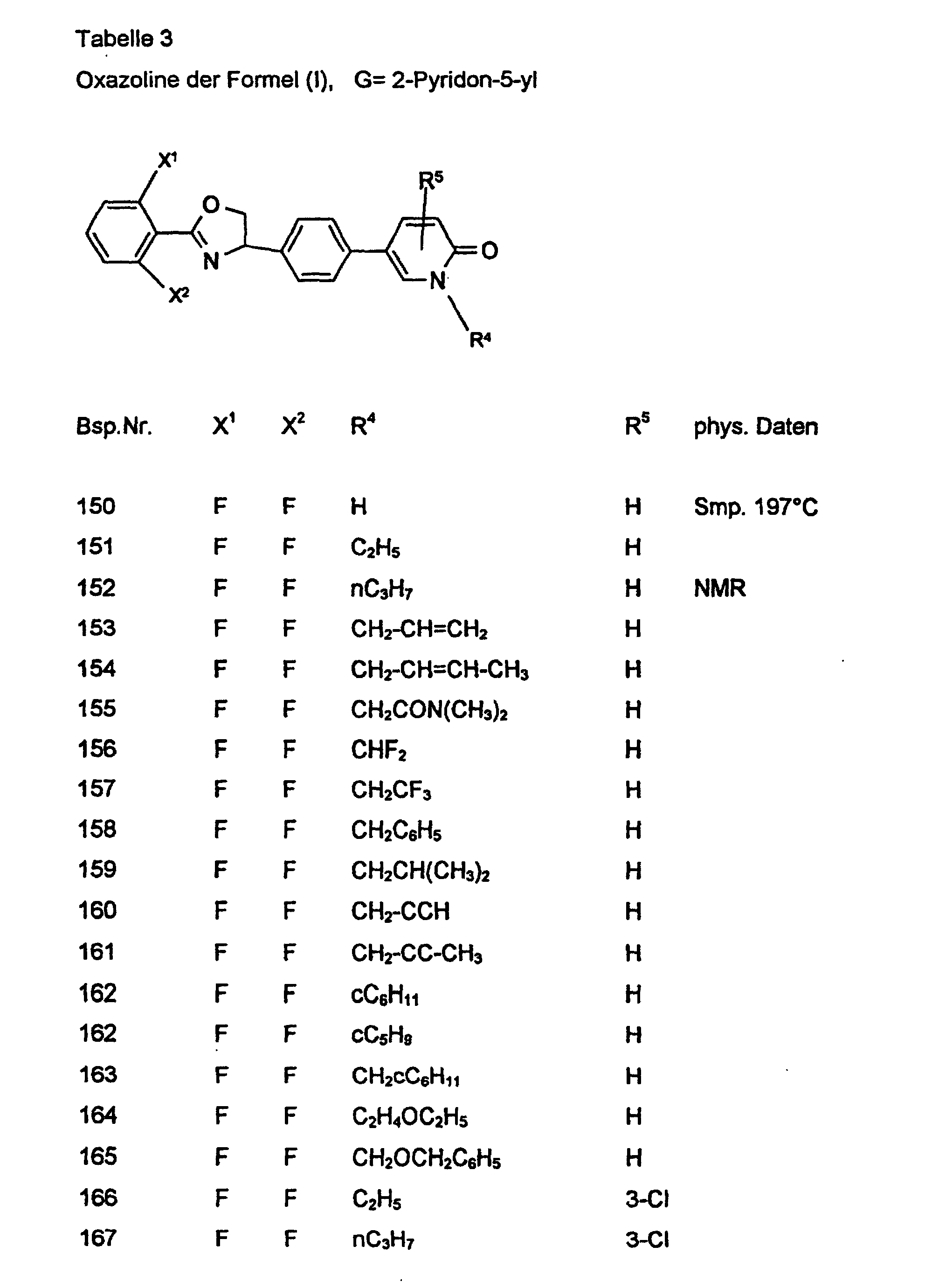

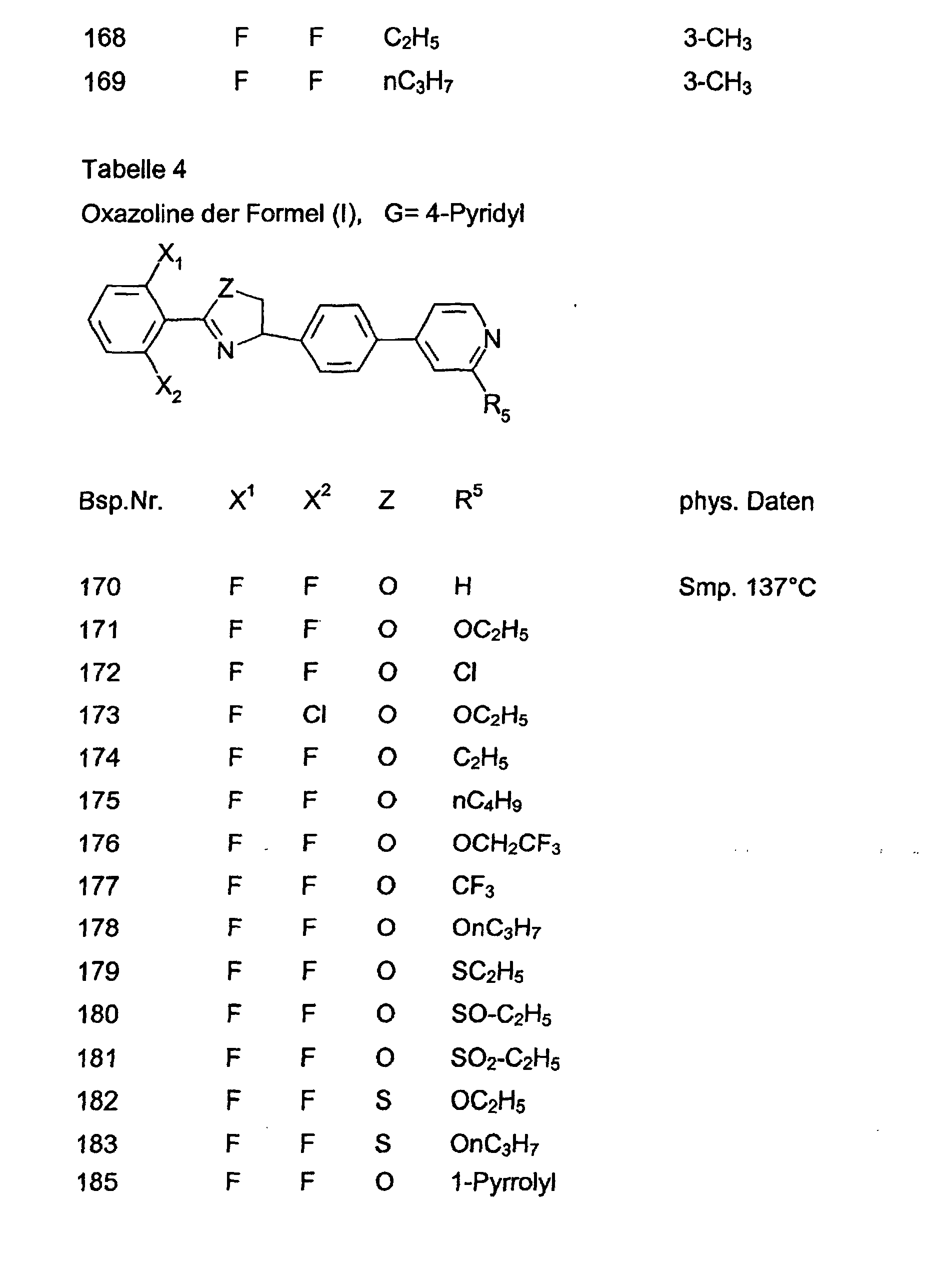

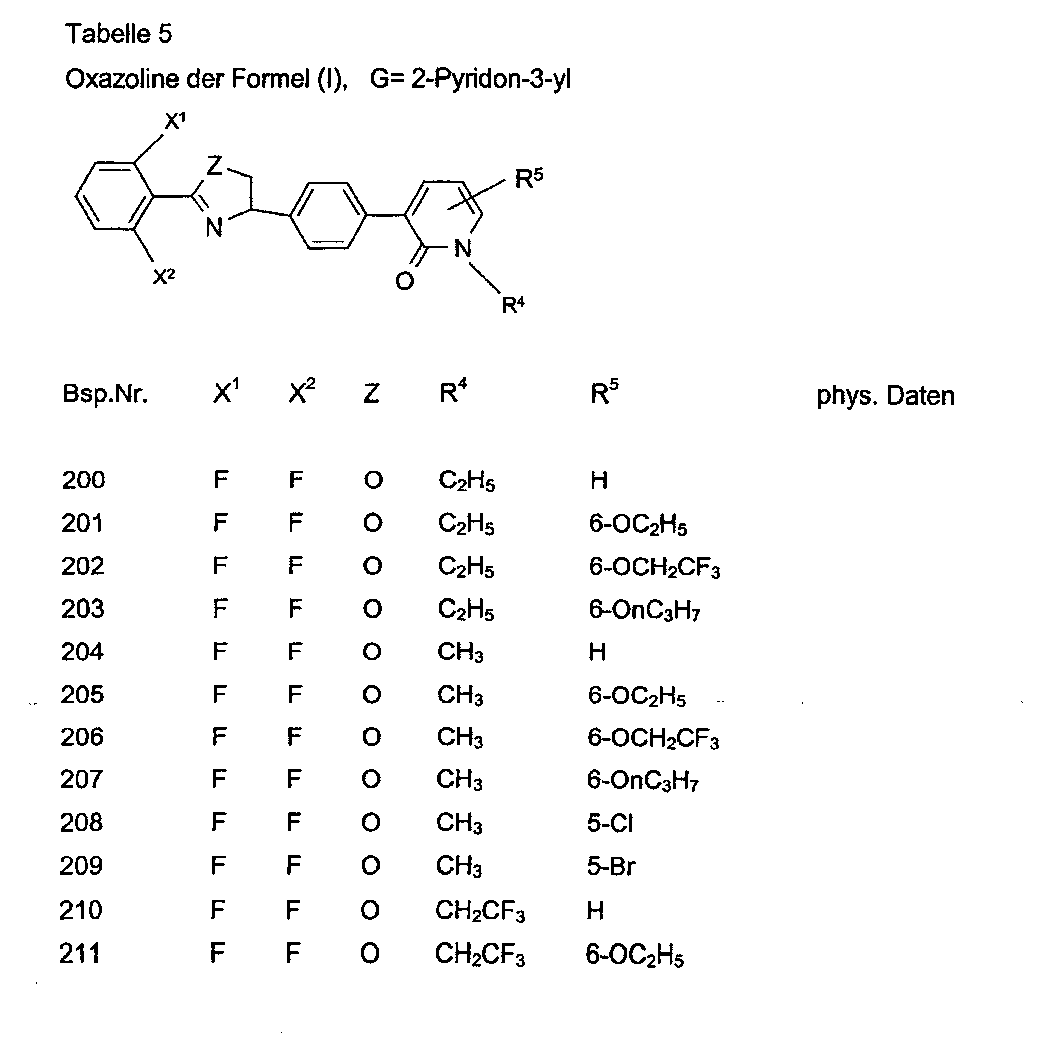

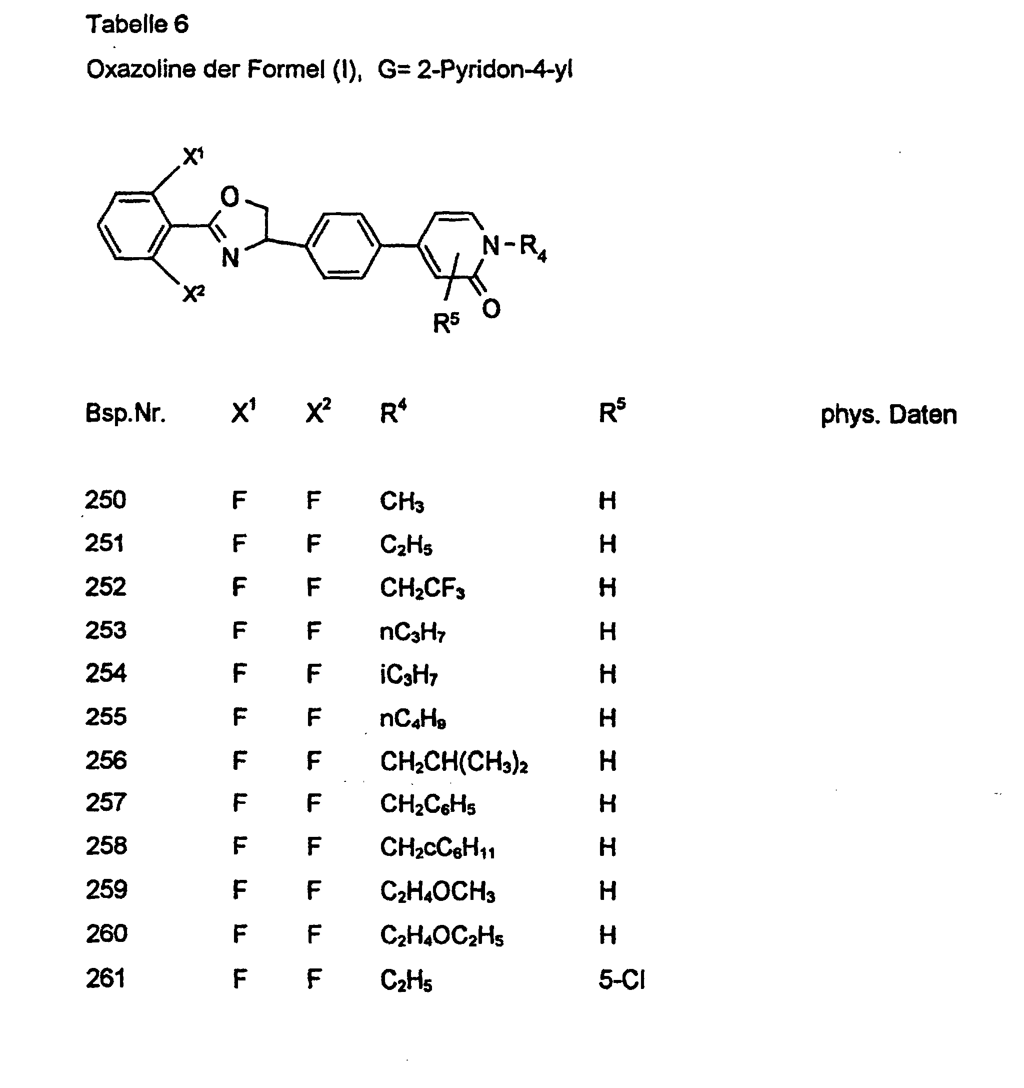

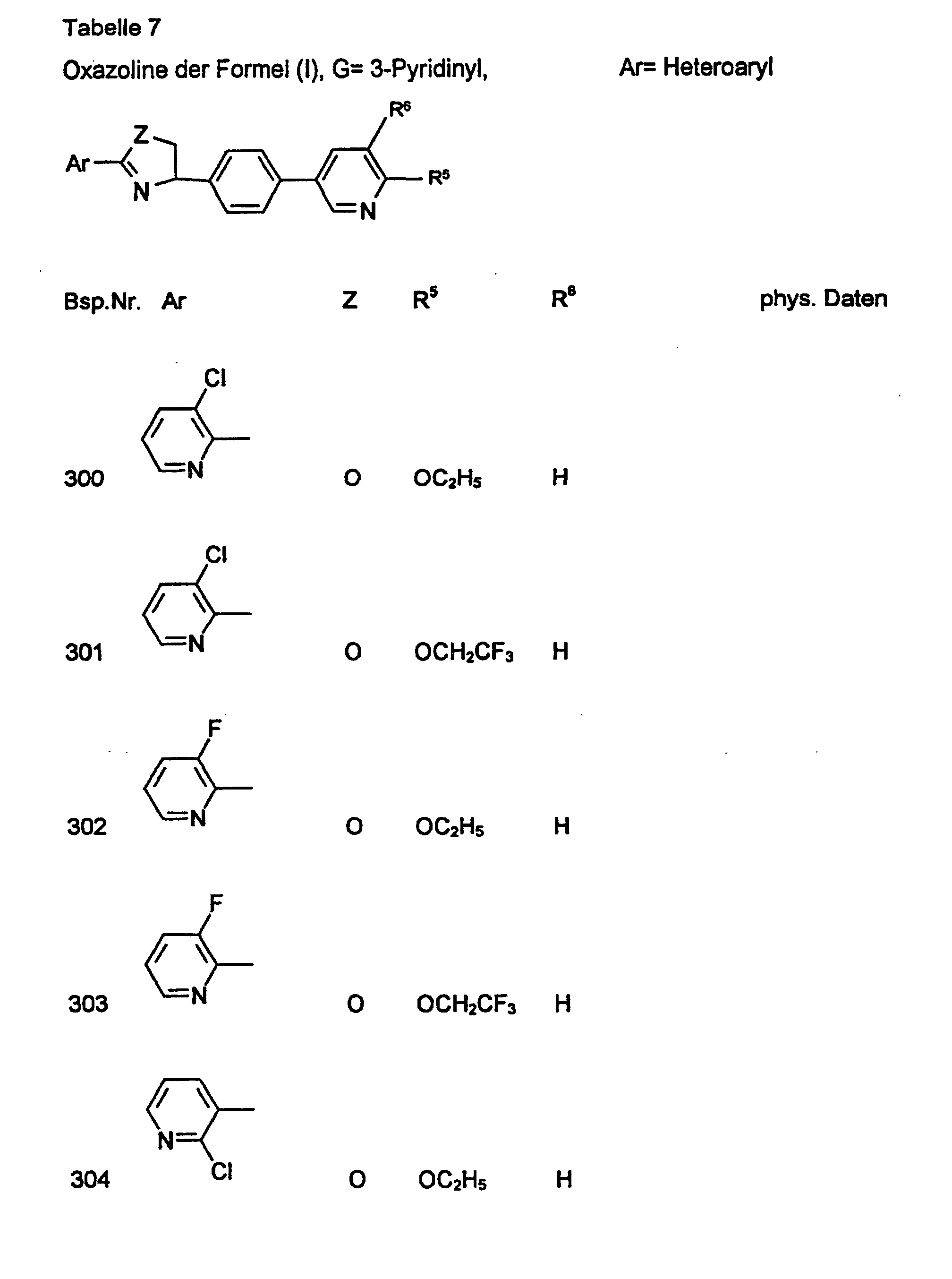

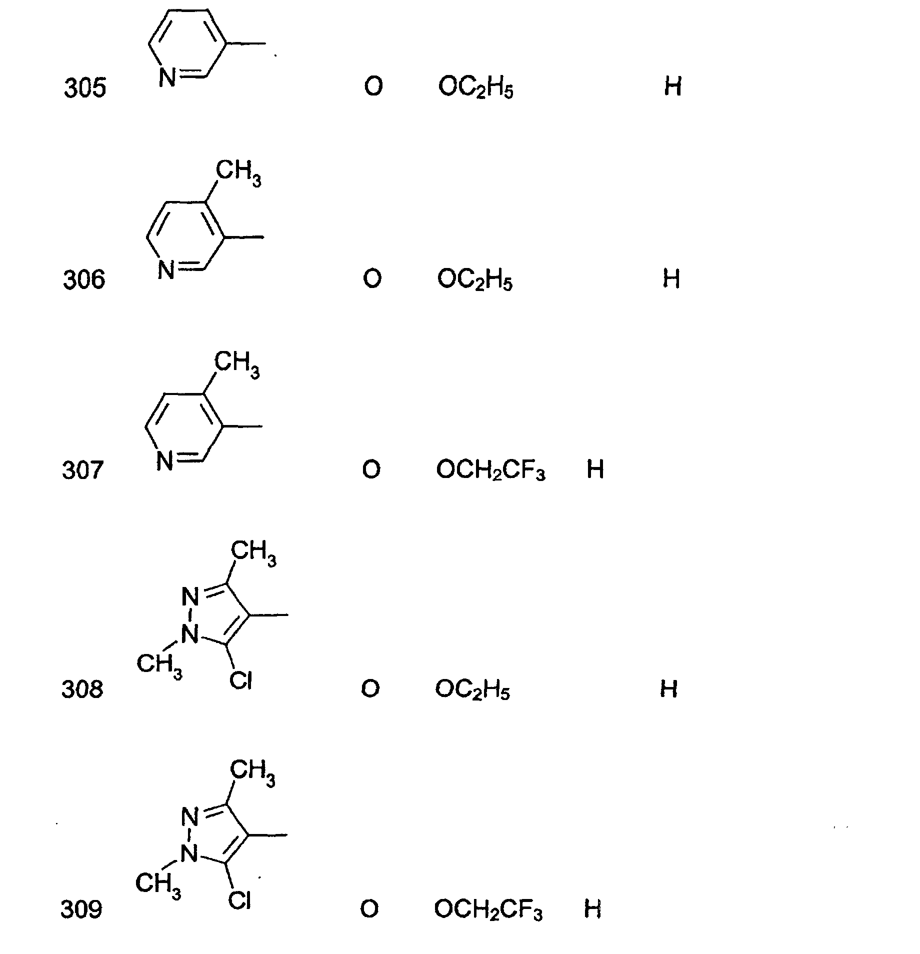

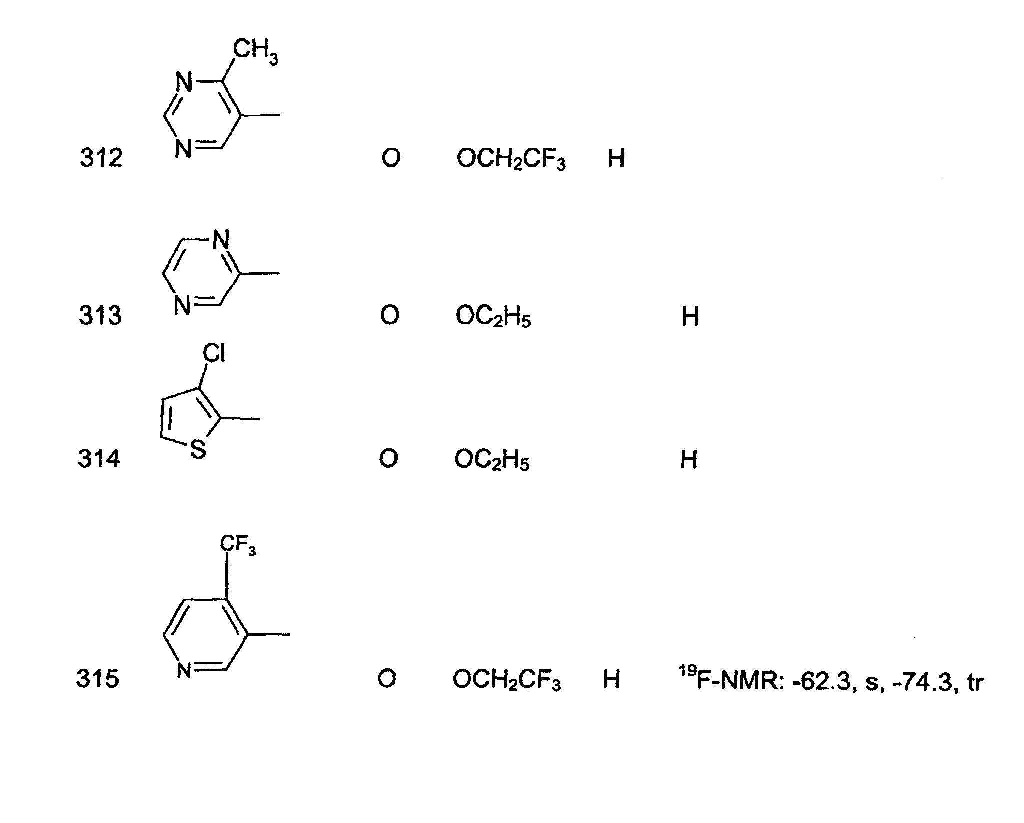

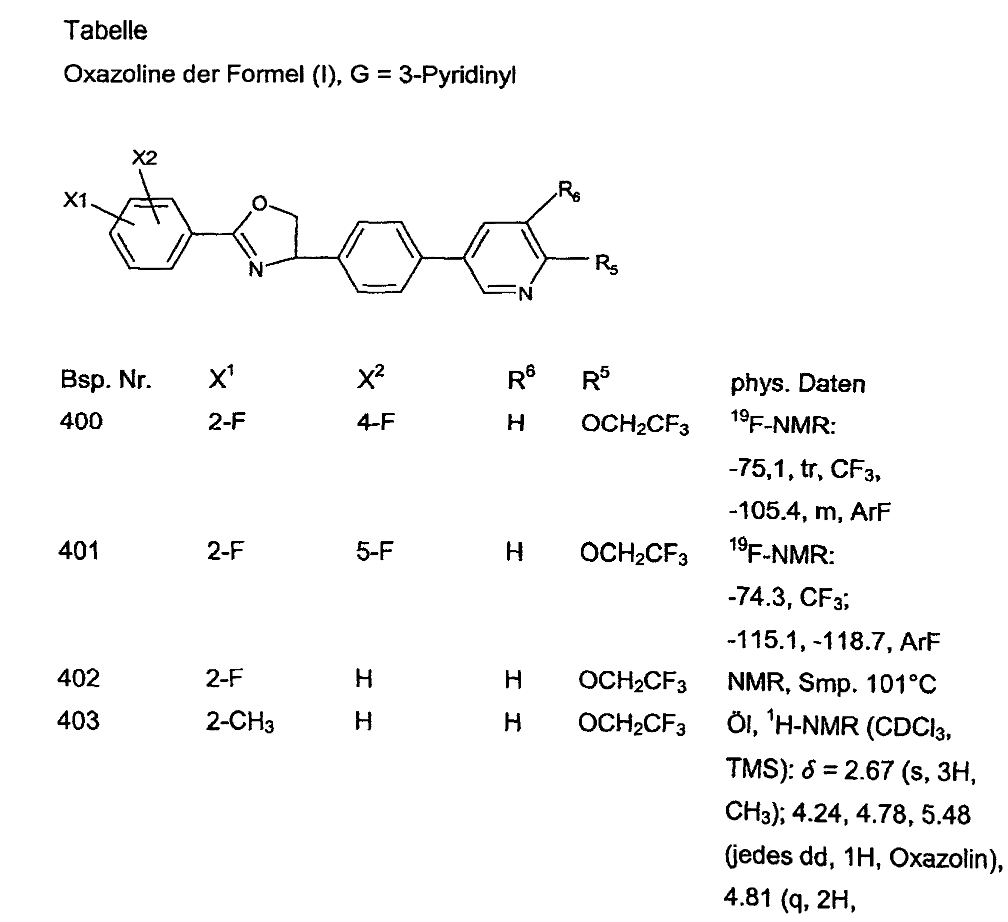

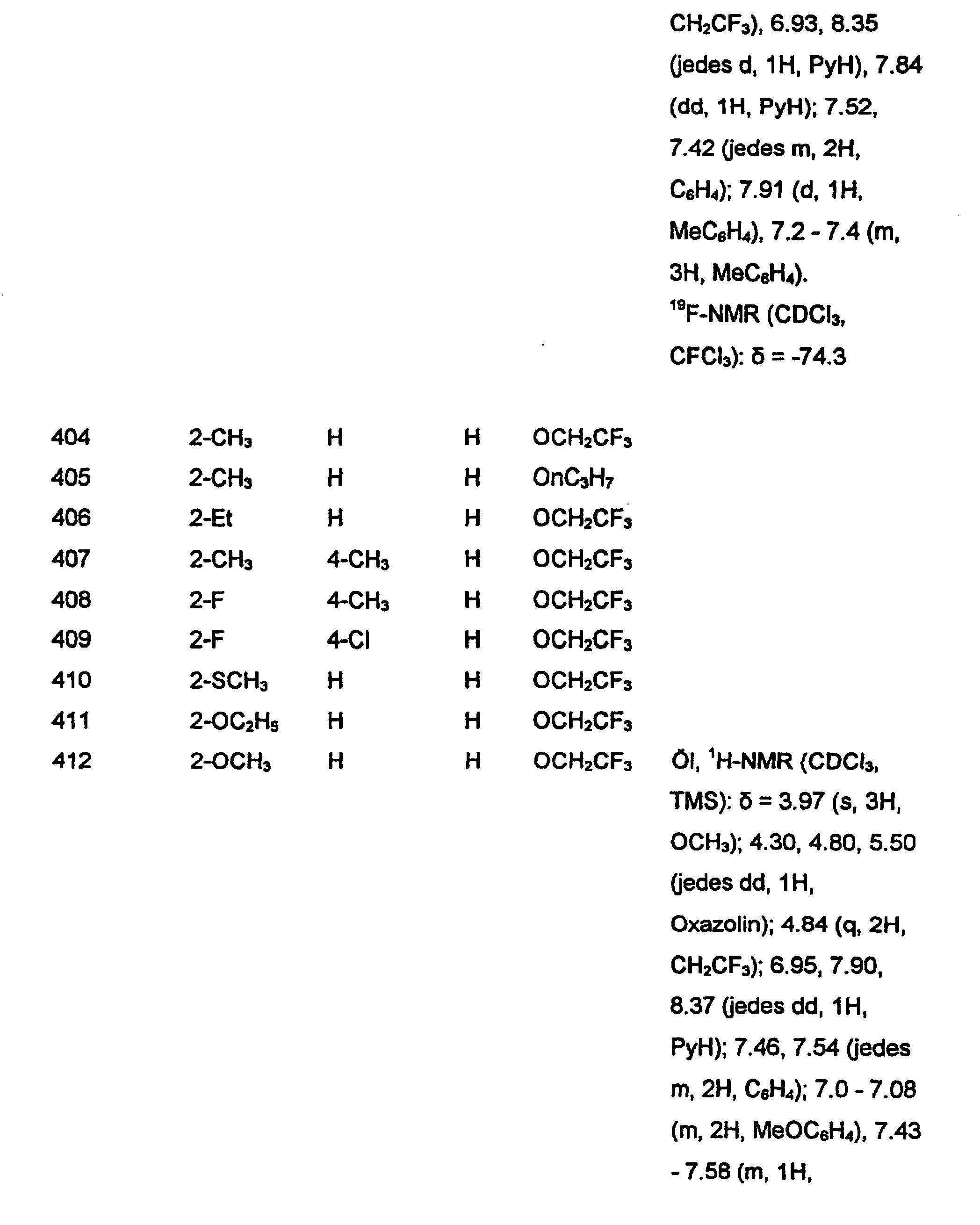

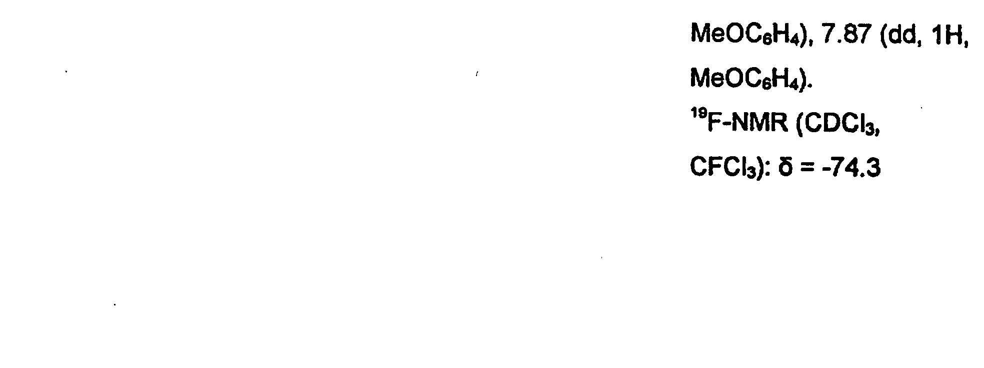

### B. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoytmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

### Beispiel 1 : Wirkung auf die Spinnmilben Tetranychus urticae

Mit einer Vollpopulation von Spinnmilben (Tetranychus urticae) stark befallene Bohnenpflanzen (Phaseolus vulgaris) wurden mit einer wäßrigen Lösung des zu prüfenden und formulierten Präparates bis zum beginnenden Abtropfen besprüht. Die Mortalität aller Milbenstadien wurde nach 7 Tagen bestimmt. Bei einer Konzentration von 300 ppm (bezogen auf den Gehalt an Wirkstoff) zeigten die Präparate gemäß Beispiel Nr. 1, 2, 3, 4, 7, 8, 9, 14, 18, 21, 31, 40, 41, 43, 44, 47, 106, 152, und 153 eine 90-100 %ige Mortalität.

### Beispiel 2 : Wirkung auf die Blattlaus Aphis fabae

Mit schwarzer Bohnenblattlaus (Aphis fabae) stark besetzte Ackerbohnen (Vicia faba) wurden mit einer wäßrigen Lösung des zu prüfenden und formulierten Präparates bis zum beginnenden Abtropfen besprüht. Die Mortalität der Blattläuse wurde nach 3 Tagen bestimmt. Bei einer Konzentration von 300 ppm (bezogen auf den Gehalt an Wirkstoff) zeigten die Präparate gemäß Beispiel Nr. 1, 2, 3, 4, 7, 8, 152 eine 90-100 %ige Mortalität.

### Beispiel 3 : Wirkung auf die Schmetterlingslarve Spodoptera litoralis

Zehn L4-Larven des Ägyptischen Baumwollwurms (Spodoptera litoralis) wurden in eine Petrischalen gesetzt, deren Boden mit Filterpapier belegt war und ca. 5 ml Nährmedium enthielt. Filterpapier, Nährmedium und eingesetzte Larven wurden dann mit einer wäßrigen Lösung des zu prüfenden und formulierten Präparates besprüht. Anschließend wurde die Petrischale mit einem Deckel verschlossen. Nach 4 Tagen Lagerung bei ca. 23 °C wurde die Wirkung des Präparates auf die Larven festgestellt. Bei einer Konzentration von 300 ppm (bezogen auf den Gehalt an Wirkstoff) bewirkten die Präparate gemäß Beispiel Nr. 3, 4, 22, 105, 106 eine 90-100 %ige Mortalität der Larven.

### Beispiel 4: Wirkung auf das Ei-Larven-Stadium von Heliothis virescens

Eine Petrischale, deren Boden mit Filterpapier belegt war und ca. 5 ml Nährmedium enthielt, wurde vorbereitet. Filterpapierstücke mit ca. 30, 24 Stunden alten Eier der Amerikanischen Tabakknospeneule (Heliothis virescens) wurden für 5 Sekunden in einer wäßrigen Lösung des zu prüfenden und formulierten Präparates getaucht und anschließend in der Petrischale ausgelegt. Weitere 200 µl der wäßrigen Lösung wurden über das Nährmedium verteilt. Nach dem Verschließen der Petrischale wurde diese bei ca. 25 °C in einer Klimakammer gelagert. Nach 6 Tagen Lagerung wurde die Mortalität des Präparates auf die Eier und die evtl. hieraus geschlüpften Larven festgestellt. Bei einer Konzentration von 300 ppm (bezogen auf den Gehalt an Wirkstoff) bewirkten die Präparate gemäß Beispiel Nr. 1, 2, 3, 4, 5, 6, 7, 8, 14, 17, 21, 22, 23, 34, 35, 39, 40, 43, 44, 106, eine 90-100 %ige Mortalität.

### Beispiel 5: Wirkung auf den Maiswurzelwurm Diabrotica undecimpunctata

Maissaatgut wurde unter Wasser 6 Stunden vorgekeimt, danach in 10 ml Glasröhrchen gegeben und mit je 2 ml Erde abgedeckt. Nach Zugabe von 1 ml Wasser blieben die Pflanzen bei 21 °C in den Glasröhrchen stehen bis zum Erreichen einer Wuchshöhe von ca. 3 cm. Dann wurden jeweils 10 L2-Larven des Maiswurzelwurms (Diabrotica undecimpunctata) in die Gläschen auf die Erde gegeben. Zwei Stunden danach wurde 1 ml einer wäßrigen Lösung des zu prüfenden und formulierten Präparates auf die Erdoberfläche in die Gläschen pipettiert. Nach 5 Tagen Standzeit unter Laborbedingungen bei 21 °C wurden Erde und Wurzelteile auf lebende Diabrotica-Larven durchsucht und die Mortalität festgestellt. Bei einer Konzentration von 300 ppm (bezogen auf den Gehalt an Wirkstoff) zeigten die Präparate gemäß Beispiel Nr. 2, 3, 5, 9, 19, 39, eine, 100 %ige Mortalität bei den eingesetzten Versuchstieren.

### Beispiel 6 : Wirkung gegen die Larven der Schafschmeißfliege Lucilia cuprina

In einem Probengefäß wurden 20 frisch geschlüpfte Larven der Schafschmeißfliege, Lucilia cuprina, auf ein Larvennährmedium aus gemahlenem Schaffleisch gesetzt, welches die Prüfsubstanz in einer Konzentration von 100 ppm enthielt. Das Wachstum der Larven auf dem Nährmedium wird über 72 Stunden bis zur Larve 3 verfolgt. Bei einer Konzentration von 100 ppm (bezogen auf den Gehalt an Wirkstoff) zeigten die Präparate gemäß Beispiel Nr. 2, 3, 4, 5, 105, 106 eine 100%ige Mortalität bei den eingesetzten Fliegenlarven.

### Beispiel 7 : Wirkung gegen Entwicklungsstadien des Katzenflohs, Ctenocephalides felis

Dem Larvennährmedium des Katzenflohs, bestehend aus Blutmehl und Quarzsand zu gleichen Teilen, wurde die Prüfsubstanz in einer Konzentration von 1000 ppm zugemischt. Auf das Medium wurden ca. 30 aus einer Zuchtkolonie frisch erhaltene Floheier verbracht. Zur Bewertung des Präparateffektes wurde der Schlupf der Flohlarven und deren Entwicklung zu Puppen und adulten Flöhen beobachtet. Die Prüfsubstanzen gemäß Beispiel Nr. 2, 3, 4 bewirkten eine Mortalität von 100 % .

## Patentansprüche

1. 1,3-Oxazolin und 1,3-Thiazolin-Derivate der Formel (I), 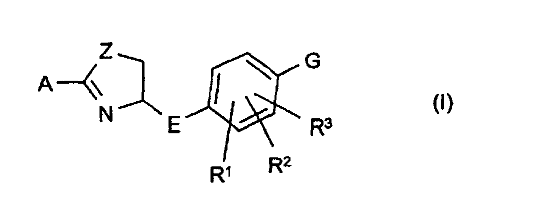 worin die Symbole folgende Bedeutungen haben:
A ist Phenyl, Pyridyl, Pyrimidinyl, Pyrazinyl, Pyrazolyl oder Thienyl, wobei jede der genannten Gruppen gegebenenfalls durch einen oder mehrere Reste X substituiert ist;
X ist gleich oder verschieden
a) Halogen, Cyano, Nitro;
b) (C₁-C₄)-Alkyl, (C₂-C₄)-Alkenyl, (C₂-C₄)-Alkinyl, (C₁-C₄)-Alkoxy, (C₁-C₄)-Alkylthio, (C₁-C₄)-Alkylsulfinyl, (C₃-C₈)-Cycloalkyl, (C₄-C₈)-Cycloalkenyl, Phenyl,
wobei die Reste der Gruppe b gegebenenfalls durch einen oder mehrere Reste aus der Gruppe Halogen, Cyano, (C₁-C₄)-Alkyl, (C₁-C₄)-Haloalkyl, (C₁-C₄)-Alkoxy und (C₁-C₄)-Haloalkoxy substituiert sind;
E ist eine Einfachbindung,
G ist ein Rest aus der Gruppe: 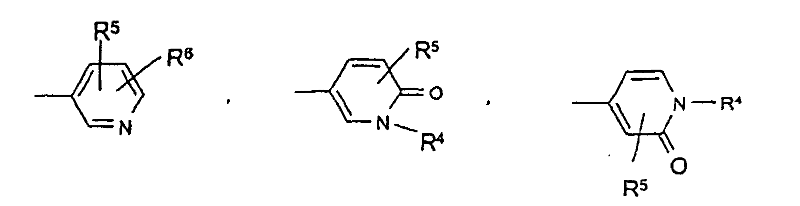 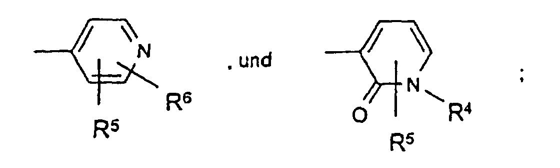
Z ist Sauerstoff oder Schwefel;
R¹, R² und R³ sind gleich oder verschieden Wasserstoff, Halogen, (C₁-C₄)Haloalkyl, (C₁-C₄)Alkyl, (C₁-C₄)Alkoxy, (C₁-C₄)Haloalkoxy oder Cyano;
R⁴ ist Wasserstoff oder eine Gruppe (C₁-C₈)Alkyl, (C₂-C₈)Alkenyl, (C₂-C₈)Alkinyl, (C₃-C₈)Cycloalkyl, (C₃-C₈)Cycloalkenyl, (C₄-C₁₀)Cycloalkyl-alkyl oder (C₇-C₁₂)Phenylalkyl, wobei jede der genannten Gruppen gegebenenfalls durch einen oder mehrere Substituenten aus der Gruppe Halogen, (C₁-C₄)Alkyl, (C₁-C₃)Haloalkyl, (C₁-C₄)Alkoxy, (C₁-C₃)Haloalkoxy, (C₁-C₃)Alkylthio, (C₃-C₈)Cycloalkyl, (C₇-C₁₂)Phenylalkoxy, (C₂-C₄)Alkylcarbonyl, Alkoxycarbonyl, (C₂-C₆)Monoalkyl- und (C₃-C₉)Dialkyl-aminocarbonyl Cyano und Tri(C₁-C₄)alkylsilyl substituiert ist;
R⁵, R⁶ sind a) Wasserstoff, Halogen, Cyano;
b) (C₁-C₈)Alkyl, (C₃-C₈)Cycloalkyl, (C₄-C₈)Cycloalkenyl, (C₁-C₈)Alkylthio, (C₁-C₈)Alkylsulfinyl, (C₁-C₈)Alkylsulfonyl, (C₇-C₁₂)Phenylalkyl, (C₇-C₁₂)Phenylalkoxy, (C₄-C₇)Oxycycloalkyl oder (C₄-C₇)Oxacycloalkenyl, welche gegebenenfalls durch einen oder mehrere Reste aus der Gruppe Halogen, (C₁-C₄)Alkyl, (C₁-C₃)Haloalkyl, (C₁-C₃)Alkoxy, (C₁-C₃)Haloalkoxy, (C₁-C₃)Alkylthio und Cyano substituiert sind; oder
c) (C₁-C₈)Alkoxy, welches gegebenenfalls durch einen oder mehrere Reste aus der Gruppe Halogen, (C₁-C₃)Alkylthio und Cyano substituiert ist.

2. Verbindung der Formel (I) nach Anspruch 1, wobei die Symbole folgende Bedeutungen haben:
A ist Phenyl oder Pyridyl;
X ist
a) Halogen, Cyano, Nitro oder
b) (C₁-C₄)-Alkyl, (C₁-C₄)-Alkenyl, (C₁-C₄)-Alkinyl, (C₁-C₄)-Alkoxy, (C₁-C₄)-Alkylthio, (C₁-C₄)-Aklylsulfinyl, (C₃-C₇)-Cycloalkyl, (C₄-C₈)-Cycloalkenyl,
wobei die Reste der Gruppe b gegebenenfalls durch einen oder mehrere Reste aus der Gruppe Halogen, Cyano, (C₁-C₄)-Alkoxy und (C₁-C₄)-Haloalkoxy substituiert sind;
E ist eine Einfachbindung ;
G ist 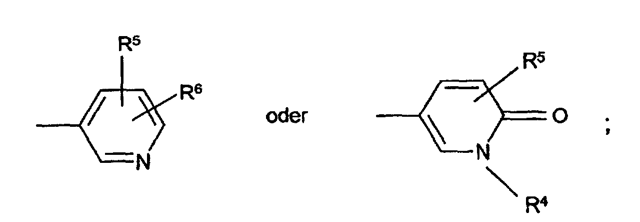
Z ist Sauerstoff;
R¹, R², R³ sind H, Halogen, (C₁-C₄)-Haloalkyl, (C₁-C₄)-Alkyl, (C₁-C₄)-Alkoxy, (C₁-C₄)-Haloalkoxy oder Cyano;
R⁴ ist H oder (C₁-C₈)-Alkyl, welches gegebenenfalls durch einen oder mehrere Reste aus der Gruppe Halogen, (C₁-C₄)-Alkyl, (C₁-C₃)-Haloalkyl, (C₁-C₄)- Alkoxy, (C₁-C₃)-Haloalkoxy, (C₁-C₃)-Alkylthio, (C₃-C₈)-Cycloalkyl, Cyano oder Tri(C₁-C₄)alkylsilyl substituiert ist;
R⁵, R⁶ sind
a) H, Halogen, Cyano;
b) (C₁-C₈)-Alkyl, (C₃-C₈)-Cycloalkyl, (C₄-C₈)-Cycloalkenyl, (C₁-C₈)- Alkylthio, (C₁-C₈)-Alkylsulfinyl, (C₁-C₈)-Alkylsulfonyl, (C₇-C₁₂)- Phenylkalkyl, (C₇-C₁₂)-Phenylalkoxy, (C₄-C₇)-Oxycycloalkyl oder (C₄-C₇)-Oxacycloalkenyl,
wobei die Gruppen b gegebenenfalls durch einen oder mehrere Reste aus der Gruppe Halogen, (C₁-C₄)-Alkyl, (C₁-C₃)-Haloalkyl, (C₁-C₃)-Alkoxy, (C₁-C₃)-Haloalkoxy, (C₁-C₃)-Alkylthio und Cyano substituiert sind; oder
c) (C₁-C₈)-Alkoxy, welches gegebenenfalls durch einen oder mehrere Reste aus der Gruppe Halogen, (C₁-C₃)Alkylthio und Cyano substituiert ist.

3. Verbindung der Formel (I) nach Anspruch 1 oder 2, wobei die Symbole folgende Bedeutungen haben:
A ist Phenyl;
X ist Halogen, (C₁-C₄)-Alkyl, (C₁-C₃)-Haloalkyl, (C₁-C₄)-Alkoxy oder (C₁-C₃)-Haloalkoxy;
E ist eine Einfachbindung;
G ist 3-Pyridyl;
Z ist Sauerstoff;
R¹, R², R³ sind H, Halogen, (C₁-C₄)-Haloalkyl, (C₁-C₄)-Alkyl, (C₁-C₄)-Alkoxy, (C₁-C₄)-Haloalkoxy;
R⁴ ist H oder (C₁-C₈)-Alkyl, welches gegebenenfalls durch einen oder mehrere Reste aus der Gruppe Halogen, (C₁-C₄)-Alkyl, (C₁-C₃)-Haloalkyl, (C₁-C₄)-Alkoxy, (C₁-C₃)-Haloalkoxy, (C₁-C₃)-Alkylthio, (C₃-C₈)-Cycloalkyl, Cyano oder Tri(C₁-C₄)alkylsilyl substituiert ist;
R⁵, R⁶ sind
a) H, Halogen, Cyano,
b) (C₁-C₈)-Alkyl, (C₁-C₈)-Alkoxy, (C₃-C₈)-Cycloalkyl, (C₄-C₈)-Cycloalkenyl, (C₁-C₈)-Alkylthio, (C₁-C₈)-Alkylsulfinyl, (C₁-C₈)-Alkylsulfonyl, (C₇-C₁₂)-Phenylkalkyl oder (C₇-C₁₂)-Phenylalkoxyl,
wobei die Gruppen b gegebenenfalls durch einen oder mehrere Reste aus der gruppe Halogen, (C₁-C₄)-Alkyl, (C₁-C₃)-Haloalkyl und Cyano substituiert sind.

4. Verbindungen der Formel (I) nach einem oder mehreren der Ansprüche 1 bis 3, ausgewählt aus den Gruppen (Ia) bis (Ie): 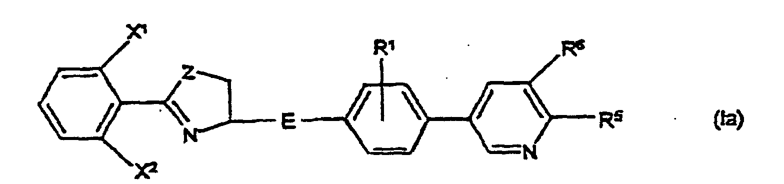 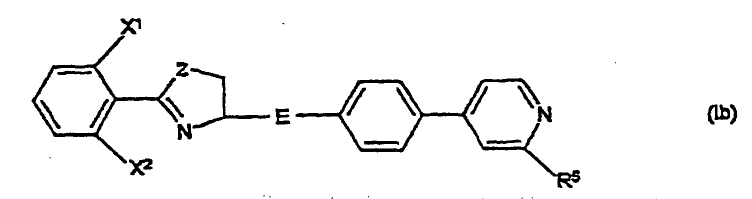 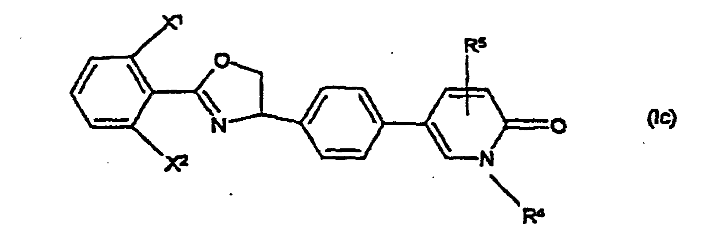 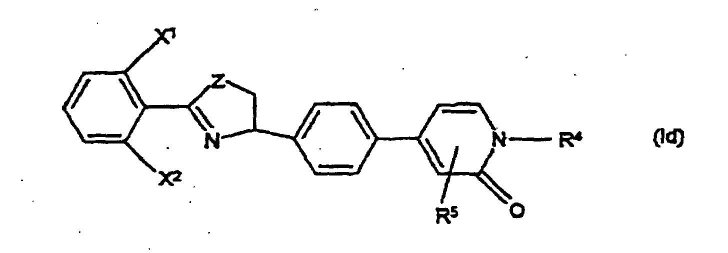 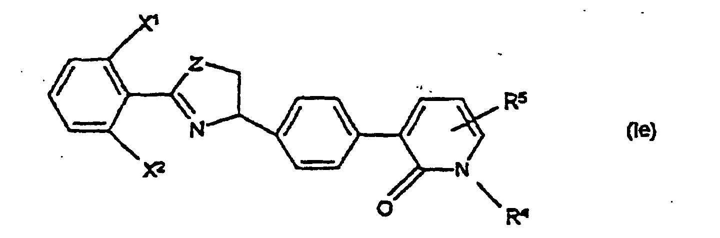 wobei die Symbole die in der Formel (I) in den Ansprüchen 1 bis 3 angegebenen Bedeutungen haben.

5. Verbindungen der Formel (I) nach Anspruch 4, ausgewählt aus der Gruppe (Ia1) bis (Ia4); 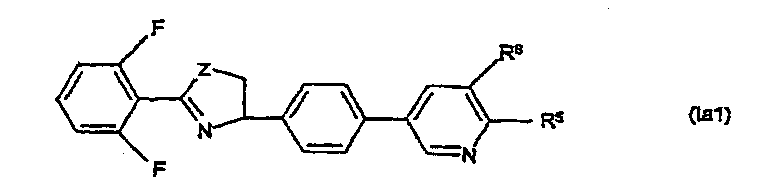 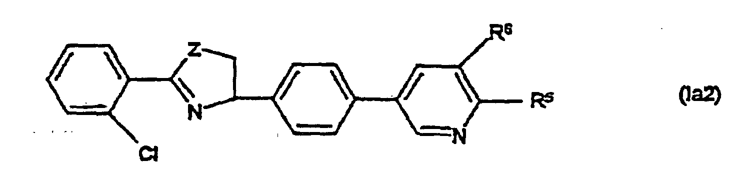 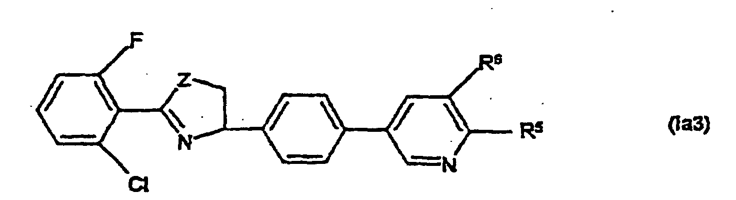 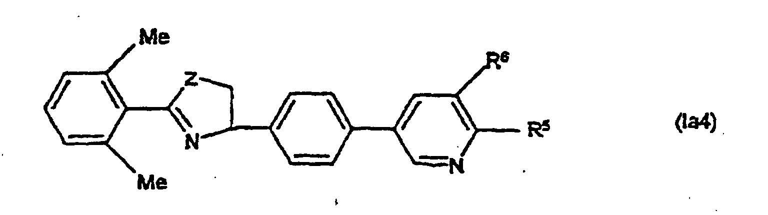 wobei die Symbole die in der Formel (I) in den Ansprüchen 1 bis 3 angegebenen Bedeutungen haben.

6. Verfahren zur Herstellung von Verbindungen der Formel (I) nach einem oder mehreren der Ansprüche 1 bis 5, **dadurch gekennzeichnet, daß** man eine Halogenund Perfluoralkylsulfonat-Verbindung der Formel (II), 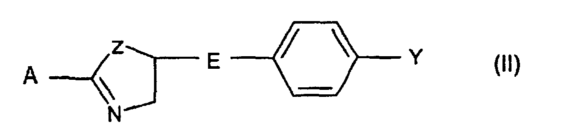 worin
Y Cl, Br, I oder Perfluoralkylsulfonat bedeutet und
A, Z und E die in der Formel (I) angegebenen Bedeutungen haben,
unter Palladiumkatalyse mit einer metallorganische Verbindung der Formel (III),
G-M (III)
worin
M eine B, Sn oder Zn-haltige Abgangsgruppe bedeutet und
G die in der Formel (I) in Anspruch (1) angegebenen Bedeutungen hat,
umsetzt.

7. Pflanzenschutzmittel, enthaltend mindestens eine Verbindung gemäß einem der Ansprüche 1 bis 5 und mindestens ein Formulierungsmittel.

8. Insektizides, akarizides oder nematizides Mittel gemäß Anspruch 7, enthaltend eine wirksame Menge mindestens einer Verbindung gemäß einem der Ansprüche 1 bis 5 zusammen mit den für diese Anwendung üblichen Zusatz- oder Hilfsstoffen.

9. Pflanzenschutzmittel, enthaltend eine insektizid, akarizid oder nematizid wirksame Menge mindestens einer Verbindung gemäß einem der Ansprüche 1 bis 5 und mindestens einem weiteren Wirkstoff zusammen mit den für diese Anwendung üblichen Hilfs- und Zusatzstoffen.

10. Mittel zur Anwendung im Holzschutz oder als Konservierungsmittel in Dichtmassen, in Anstrichfarben, in Kühlschmiermitteln für die Metallbearbeitung oder in Bohr- und Schneidölen, enthaltend eine wirksame Menge mindestens einer Verbindung gemäß einem der Ansprüche 1 bis 5 zusammen mit den für diese Anwendungen üblichen Hilfs- und Zusatzstoffen.

11. Verbindung gemäß einem der Ansprüche 1 bis 5 oder Mittel gemäß Anspruch 7, 8 oder 9 zur Anwendung als Tierarzneimittel.

12. Verfahren zur Herstellung eines Mittels gemäß einem der Ansprüche 7 bis 10, **dadurch gekennzeichnet, daß** man den Wirkstoff und die weiteren Zusätze zusammen gibt und in eine geeignete Anwendungsform bringt.

13. Verwendung einer Verbindung gemäß einem der Ansprüche 1 bis 5 oder eines Mittels gemäß einem der Ansprüche 7, und 10 als Holzschutzmittel oder als Konservierungsmittel in Dichtmitteln, in Anstrichfarben, in Kühlschmiermitteln für die Metallbearbeitung oder in Bohr- und Schneideölen.

14. Verwendung von Verbindungen gemäß einem der Ansprüche 1 bis 5 oder eines Mittels gemäß einem der Ansprüche 7, 8 oder 9 zur Bekämpfung von Schadinsekten, Acarina, Mollusken und Nematoden.

15. Verfahren zur Bekämpfung von Schadinsekten, Acarina, Mollusken und Nematoden, bei welchem man auf diese oder die von ihnen befallenen Pflanzen, Flächen oder Substrate eine wirksame Menge einer Verbindung gemäß einem der Ansprüche 1 bis 5 oder eines Mittels gemäß einem der Ansprüche 7, 8 oder 9 appliziert.

16. Saatgut, behandelt oder beschichtet mit einer wirksamen Menge einer Verbindung gemäß einem der Ansprüche 1 bis 5 oder eines Mittels gemäß einem der Ansprüche 7, 8 oder 9.

## Claims

1. A 1,3-Oxazoline derivative. and a 1,3-thiazoline derivative of the formula (I) 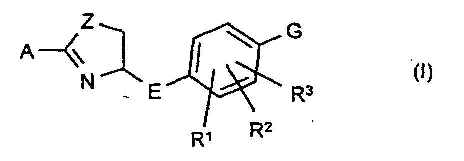 in which the symbols have the following meanings:
A is phenyl, pyridyl, pyrimidinyl, pyrazinyl, pyrazolyl or thienyl, each of the abovementioned groups optionally being substituted by one or more radicals X;
X is identical or different
a) halogen, cyano, nitro;
b) (C₁-C₄)alkyl, (C₂-C₄)alkenyl, (C₂-C₄)alkynyl, (C₁-C₄)alkoxy, (C₁-C₄)alkylthio, (C₁-C₄)alkylsulfinyl, (C₃-C₈)cycloalkyl, (C₄-C₈)cycloalkenyl, phenyl,
the radicals of group b optionally being substituted by one or more radicals selected from the group consisting of halogen, cyano, (C₁-C₄)alkyl, (C₁-C₄)-haloalkyl, (C₁-C₄)alkoxy and (C₁-C₄)halo-alkoxy;
E is a single bond;
G is a radical selected from the group consisting of: 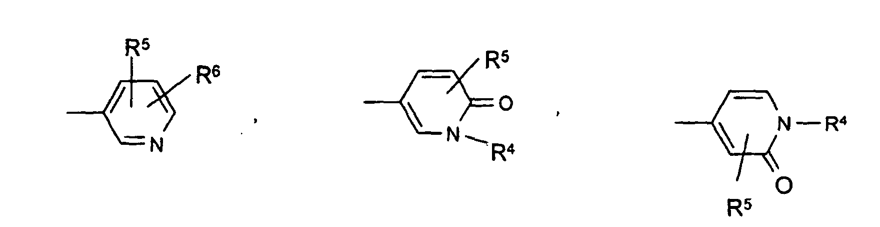 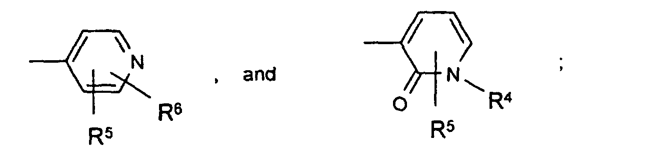
Z is oxygen or sulfur;
R¹, R² and R³ are identical or different hydrogen, halogen, (C₁-C₄)haloalkyl, (C₁-C₄)alkyl, (C₁-C₄)alkoxy, (C₁-C₄)haloalkoxy or cyano;
R⁴ is hydrogen or a group (C₁-C₈)alkyl, (C₂-C₈)alkenyl, (C₂-C₈)alkynyl, (C₃-C₈)cyclo-alkyl, (C₃-C₈)cycloalkenyl, (C₄-C₁₀)cyclo-alkylalkyl or (C₇-C₁₂)phenylalkyl, each of the abovementioned groups optionally being substituted by one or more substituents selected from the group consisting of halogen, (C₁-C₄)alkyl, (C₁-C₃)haloalkyl, (C₁-C₄)alkoxy, (C₁-C₃)haloalkoxy, (C₁-C₃)- alkylthio, (C₃-C₈)cycloalkyl, (C₇-C₁₂)phenylalkoxy, (C₂-C₄)alkylcarbonyl, alkoxycarbonyl, (C₂-C₆)monoalkyl- and (C₃-C₉)dialkylaminocarbonyl, cyano and tri(C₁-C₄)alkylsilyl;
R⁵ and R⁶ are
a) hydrogen, halogen, cyano;
b) (C₁-C₈)alkyl, (C₃-C₈)cycloalkyl, (C₄-C₈)-cycloalkenyl, (C₁-C₈)alkylthio, (C₁-C₈)-alkylsulfinyl, (C₁-C₈)alkylsulfonyl, (C₇-C₁₂)phenylalkyl, (C₇-C₁₂)phenylalkoxy, (C₄-C₇)oxycycloalkyl or (C₄-C₇)-oxacycloalkenyl, each of which is optionally substituted by one or more radicals selected from the group consisting of halogen, (C₁-C₄)alkyl, (C₁-C₃)haloalkyl, (C₁-C₃)alkoxy, (C₁-C₃)-haloalkoxy, (C₁-C₃)alkylthio and cyano; or
c) (C₁-C₈)alkoxy which is optionally substituted by one or more radicals selected from the group consisting of halogen, (C₁-C₃)alkylthio and cyano.

2. A compound of the formula (I) as claimed in claim 1, where the symbols have the following meanings:
A is phenyl or pyridyl;
X is
a) halogen, cyano, nitro or
b) (C₁-C₄)alkyl, (C₁-C₄)alkenyl, (C₁-C₄)-alkynyl, (C₁-C₄)alkoxy, (C₁-C₄)alkylthio, (C₁-C₄)alkylsulfinyl, (C₃-C₇)cycloalkyl, (C₄-C₈)cycloalkenyl,
the radicals of group b by optionally [lacuna] substituted by one or more radicals selected from the group consisting of halogen, cyano, (C₁-C₄)-alkoxy and (C₁-C₄)haloalkoxy;
E is a single bond;
G is 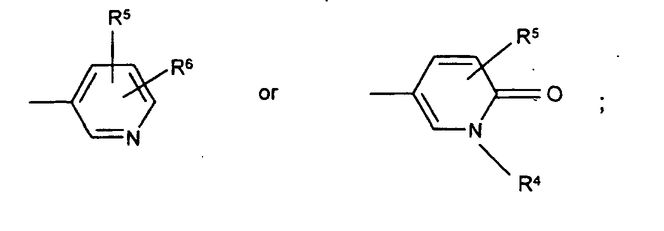
Z is oxygen;
R¹, R², R³ are H, halogen, (C₁-C₄)haloalkyl, (C₁-C₄)alkyl, (C₁-C₄)alkoxy, (C₁-C₄)haloalkoxy or cyano;
R⁴ is H or (C₁-C₈)alkyl which is optionally substituted by one or more radicals selected from the group consisting of halogen, (C₁-C₄)alkyl, (C₁-C₃)haloalkyl, (C₁-C₄)alkoxy, (C₁-C₃)haloalkoxy, (C₁-C₃)alkylthio, (C₃-C₈)-cycloalkyl, cyano or tri(C₁-C₄)alkylsilyl;
R⁵, R⁶ are
a) H, halogen, cyano;
b) (C₁-C₈)alkyl, (C₃-C₈)cycloalkyl, (C₄-C₈)-cycloalkenyl, (C₁-C₈)alkylthio, (C₁-C₈)-alkylsulfinyl, (C₁-C₈)alkylsulfonyl, (C₇-C₁₂)phenylalkyl, (C₇-C₁₂)phenylalkoxy, (C₄-C₇)oxycycloalkyl or C₄-C₇)-oxacycloalkenyl, the groups b optionally being substituted by one or more radicals selected from the group consisting of halogen, (C₁-C₄)alkyl, (C₁-C₃)haloalkyl, (C₁-C₃)alkoxy, (C₁-C₃)haloalkoxy, (C₁-C₃)alkylthio and cyano; or
c) (C₁-C₈)alkoxy which is optionally substituted by one or more radicals selected from the group consisting of halogen, (C₁-C₃)alkylthio and cyano.

3. A compound of the formula (I) as claimed in claim 1 or 2, where the symbols have the following meanings:
A is phenyl;
X is halogen, (C₁-C₄)alkyl, (C₁-C₃)haloalkyl, (C₁-C₄)alkoxy or (C₁-C₃)haloalkoxy;
E is a single bond;
G is 3-pyridyl;
Z is oxygen;
R¹, R², R³ are H, halogen, (C₁-C₄)haloalkyl, (C₁-C₄)alkyl, (C₁-C₄)alkoxy, (C₁-C₄)haloalkoxy;
R⁴ is H or (C₁-C₈)alkyl which is optionally substituted by one or more radicals selected from the group consisting of halogen, (C₁-C₄)alkyl, (C₁-C₃)haloalkyl, (C₁-C₄)alkoxy, (C₁-C₃)haloalkoxy, (C₁-C₃)alkylthio, (C₃-C₈)cycloalkyl, cyano or tri(C₁-C₄)alkylsilyl;
R⁵, R⁶ are
a) H, halogen, cyano,
b) (C₁-C₈)alkyl, (C₁-C₈)alkoxy, (C₃-C₈)cycloalkyl, (C₄-C₈)cycloalkenyl, (C₁-C₈)alkylthio, (C₁-C₈)alkylsulfinyl, (C₁-C₈)alkylsulfonyl, (C₇-C₁₂)phenylalkyl or (C₇-C₁₂)phenylalkoxy,
the groups b optionally being substituted by one or more radicals selected from the group consisting of halogen, (C₁-C₄)alkyl, (C₁-C₃)haloalkyl and cyano.

4. A compound of the formula (I) as claimed in one or more of claims 1 to 3 selected from the groups consisting of (Ia) to Ie): 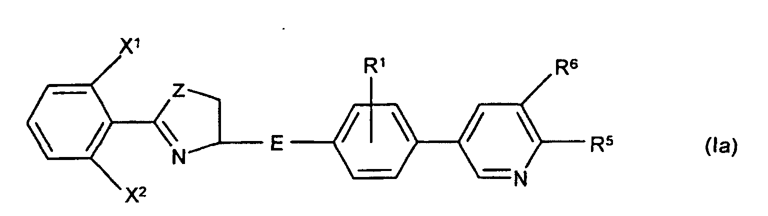 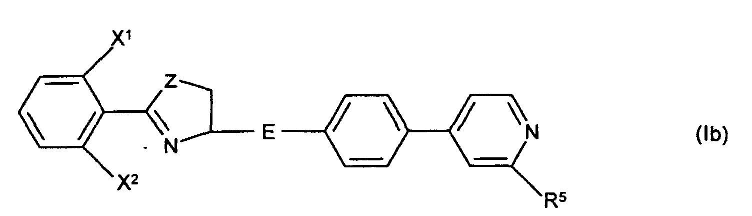 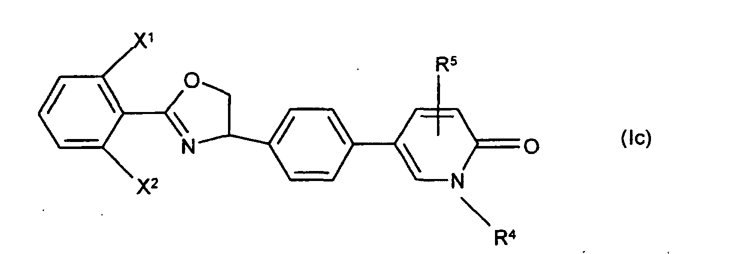 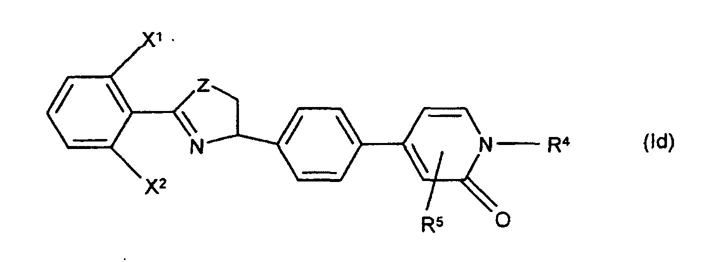 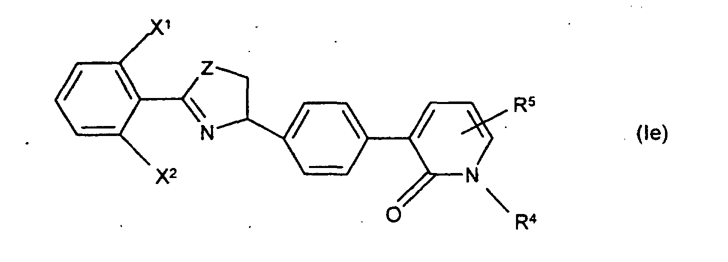 where the symbols have the meanings given in the formula (I) in claims 1 to 3.

5. A compound of the formula (I) as claimed in claim 4, selected from the group consisting of (Ia1) to (Ia4): 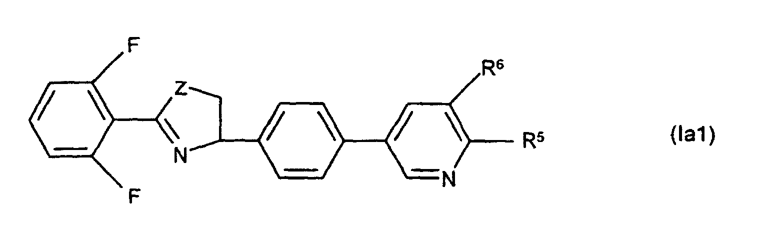 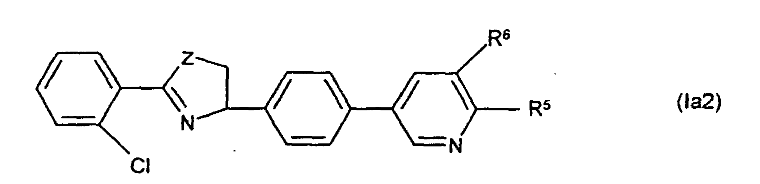 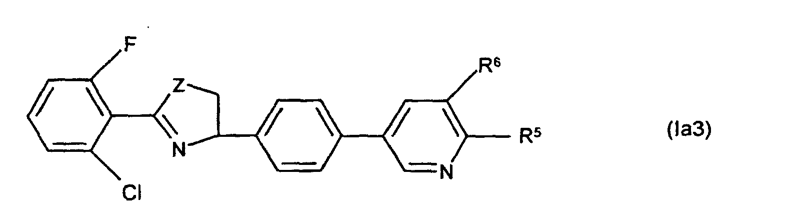 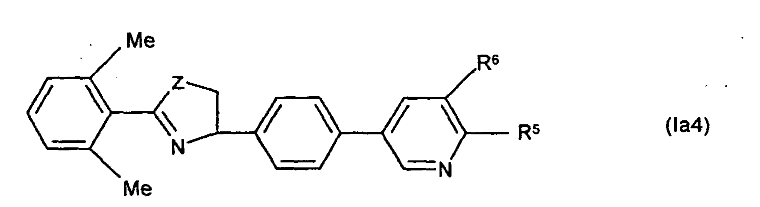 where the symbols have the meanings given in the formula (I) in claims 1 to 3.

6. A process for the preparation of compounds of the formula (I) as claimed in one or more of claims 1 to 5, which comprises reacting a halogen and perfluoroalkylsulfonate compound of the formula (II) 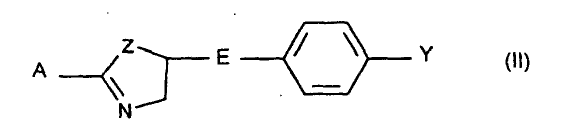 in which
Y is Cl, Br, I, I or perfluoroalkylsulfonate and
A, Z and E have the meanings given in the formula (I)
with an organometallic compound of the formula (III)
G-M (III)
in which
M is a B-, Sn- or Zn-containing leaving group and
G has the meanings given in the formula (I) in claim (I), with palladium catalysis.

7. A plant protection composition which comprises at least one compound as claimed in any of claims 1 to 5 and at least one formulation auxiliary.

8. An insecticidal, acaricidal or nematicidal composition as claimed in claim 7 which comprises an effective amount of at least one compound as claimed in any of claims 1 to 5 together with the additives or auxiliaries conventionally used for this application.

9. A crop protection composition which comprises an insecticidally, acaricidally or nematicidally effective amount of at least one compound as claimed in any of claims 1 to 5 and at least one further active substance together with the auxiliaries and additives conventionally used for this application.

10. A composition for use in the protection of timber or as preservative in sealants, in paints, in cooling lubricants for metalworking or in drilling and cutting oils, comprising an effective amount of at least one compound as claimed in any of claims 1 to 5 together with the auxiliaries and additives conventionally used for these applications.

11. A compound as claimed in any of claims 1 to 5 or a composition as claimed in claim 7, 8 or 9 for use as a veterinary medicament.

12. A process for the preparation of a composition as claimed in any of claims 7 to 10, which comprises combining the active substance and the other additives and bringing the mixture into a suitable use form.

13. The use of a compound as claimed in any of claims 1 to 5 or of a composition as claimed in either of claims 7 and 10 as timber preservative or as preservative in sealants, in paints, in cooling lubricants for metalworking or in drilling and cutting oils.

14. The use of a compound as claimed in any of claims 1 to 5 or of a composition as claimed in any of claims 7, 8 or 9 for controlling harmful insects, Acarina, mollusks and nematodes.

15. A method for controlling harmful insects, Acarina, mollusks and nematodes, in which an effective amount of a compound as claimed in any of claims 1 to 5 or of a composition as claimed in any of claims 7, 8 or 9 is applied to these or to the plants, areas or substrates infested with them.

16. Seed, coated with an effective amount of a compound as claimed in any of claims 1 to 5 or of a composition as claimed in any of claims 7, 8 or 9.

## Revendications

1. Dérivés de 1,3-oxazoline et de 1,3-thiazoline de formule (I) 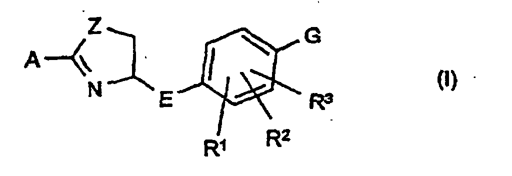 dans laquelle les symboles ont les significations suivantes:
A représente un groupe phényle, pyridyle, pyrimidinyle, pyrazinyle, pyrazolyle ou thiényle, chacun des groupes cités étant éventuellement substitué par un ou plusieurs restes X;
les X sont identiques ou différents et représentent
a) un reste halogéno, cyano ou nitro;
b) un reste alkyle en C₁-C₄, alcényle en C₂-C₄, alcynyle en C₂-C₄, alcoxy en C₁-C₄, alkylthio en C₁-C₄, alkylsulfinyle en C₁-C₄, cycloalkyle en C₃-C₈, cycloalcényle en C₄-C₈, phényle,
les restes du groupe b étant éventuellement substitués par un ou plusieurs restes du groupe constitué par les restes halogéno, cyano, alkyle en C₁-C₄, halogénoalkyle en C₁-C₄, alcoxy en C₁-C₄ et halogénoalcoxy en C₁-C₄;
E est une liaison simple,
G est un reste du groupe constitué par: 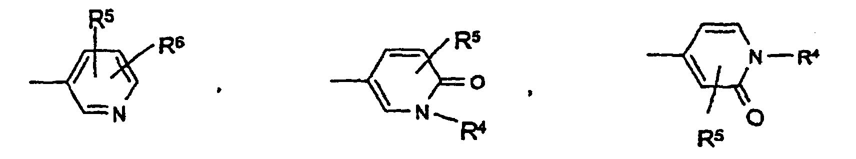 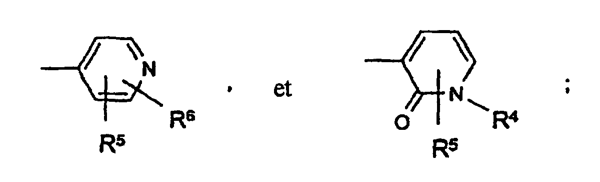
Z représente l'oxygène ou le soufre;
R¹, R² et R³ sont identiques ou différents et représentent l'hydrogène ou un reste halogéno, halogénoalkyle en C₁-C₄, alkyle en C₁-C₄, alcoxy en C₁-C₄, halogénoalcoxy en C₁-C₄ ou cyano;
R⁴ représente l'hydrogène ou un groupe alkyle en C₁-C₈, alcényle en C₂-C₈, alcynyle en C₂-C₈, cycloalkyle en C₃-C₈, cycloalcényle en C₃-C₈, cycloalkylalkyle en C₄-C₁₀ ou phénylalkyle en C₇-C₁₂, chacun des groupes cités étant éventuellement substitué par un ou plusieurs substituants du groupe constitué par les restes halogéno, alkyle en C₁-C₄, halogénoalkyle en C₁-C₃, alcoxy en C₁-C₄, halogénoalcoxy en C₁-C₃, alkylthio en C₁-C₃, cycloalkyle en C₃-C₈, phénylalcoxy en C₇-C₁₂, alkylcarbonyle en C₂-C₄, alcoxycarbonyle, monoalkylaminocarbonyle en C₂-C₆, dialkylaminocarbonyle en C₃-C₉, cyano et tri(alkyl en C₁-C₄)silyle;
R⁵, R⁶ représentent
a) l'hydrogène ou un reste halogéno ou cyano;
b) un reste alkyle en C₁-C₈, cycloalkyle en C₃-C₈, cycloalcényle en C₄-C₈, alkylthio en C₁-C₈, alkylsulfinyle en C₁-C₈, alkylsulfonyle en C₁-C₈, phénylalkyle en C₇-C₁₂, phénylalcoxy en C₇-C₁₂, oxycycloalkyle en C₄-C₇ ou oxacycloalcényle en C₄-C₇, pouvant éventuellement être substitué par un ou plusieurs restes du groupe constitué par les restes halogéno, alkyle en C₁-C₄, halogénoalkyle en C₁-C₃, alcoxy en C₁-C₃, halogénoalcoxy en C₁-C₃, alkylthio en C₁-C₃ et cyano; ou
c) un reste alcoxy en C₁-C₈ éventuellement substitué par un ou plusieurs restes du groupe constitué par les restes halogéno, alkylthio en C₁-C₃ et cyano.

2. Composé de formule (I) selon la revendication 1, dans lequel les symboles ont les significations suivantes:
A représente un groupe phényle ou pyridyle;
X représente
a) un reste halogéno, cyano ou nitro; ou
b) un reste alkyle en C₁-C₄, alcényle en C₂-C₄, alcynyle en C₂-C₄, alcoxy en C₁-C₄, alkylthio en C₁-C₄, alkylsulfinyle en C₁-C₄, cycloalkyle en C₃-C₇, cycloalcényle en C₄-C₈,
les restes du groupe b étant éventuellement substitués par un ou plusieurs restes du groupe constitué par les restes halogéno, cyano, alcoxy en C₁-C₄ et halogénoalcoxy en C₁-C₄;
E est une liaison simple,
G représente un reste 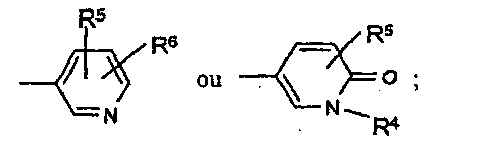
Z représente l'oxygène;
R¹, R² et R³ représentent l'hydrogène ou un reste halogéno, halogénoalkyle en C₁-C₄, alkyle en C₁-C₄, alcoxy en C₁-C₄, halogénoalcoxy en C₁-C₄ ou cyano;
R⁴ représente l'hydrogène ou un groupe alkyle en C₁-C₈ éventuellement substitué par un ou plusieurs restes du groupe constitué par les restes halogéno, alkyle en C₁-C₄, halogénoalkyle en C₁-C₃, alcoxy en C₁-C₄, halogénoalcoxy en C₁-C₃, alkylthio en C₁-C₃, cycloalkyle en C₃-C₈, cyano ou tri(alkyl en C₁-C₄)silyle;
R⁵, R⁶ représentent
b) un groupe alkyle en C₁-C₈, cycloalkyle en C₃-C₈, cycloalcényle en C₄-C₈, alkylthio en C₁-C₈, alkylsulfinyle en C₁-C₈, alkylsulfonyle en C₁-C₈, phénylalkyle en C₇-C₁₂, phénylalcoxy en C₇-C₁₂, oxycycloalkyle en C₄-C₇ ou oxacycloalcényle en C₄-C₇, les groupes b étant éventuellement substitués par un ou plusieurs restes du groupe constitué par les restes halogéno, alkyle en C₁-C₄, halogénoalkyle en C₁-C₃, alcoxy en C₁-C₃, halogénoalcoxy en C₁-C₃, alkylthio en C₁-C₃ et cyano; ou
c) un reste alcoxy en C₁-C₈ éventuellement substitué par un ou plusieurs restes du groupe constitué par les restes halogéno, alkylthio en C₁-C₃ et cyano.

3. Composé de formule (I) selon la revendication 1 ou 2, dans lequel les symboles ont la signification suivante:
A représente un groupe phényle;
X représente un reste halogéno, alkyle en C₁-C₄, halogénoalkyle en C₁-C₃, alcoxy en C₁-C₄ ou halogénoalcoxy en C₁-C₃;
E est une liaison simple,
G représente un reste 3-pyridyle;
Z représente l'oxygène;
R¹, R² et R³ représentent l'hydrogène ou un reste halogéno, halogérioalkyle en C₁-C₄, alkyle en C₁-C₄, alcoxy en C₁-C₄ ou halogénoalcoxy en C₁-C₄;
R⁴ représente l'hydrogène ou un groupe alkyle en C₁-C₈ éventuellement substitué par un ou plusieurs restes du groupe constitué par les restes halogéno, alkyle en C₁-C₄, halogénoalkyle en C₁-C₃, alcoxy en C₁-C₄, halogénoalcoxy en C₁-C₃, alkylthio en C₁-C₃, cycloalkyle en C₃-C₈, cyano ou tri(alkyl en C₁-C₄)silyle;
R⁵, R⁶ représentent
a) l'hydrogène ou un reste halogéno ou cyano,
b) un groupe alkyle en C₁-C₈, alcoxy en C₁-C₈, cycloalkyle en C₃-C₈, cycloalcényle en C₄-C₈, alkylthio en C₁-C₈, alkylsulfinyle en C₁-C₈, alkylsulfonyle en C₁-C₈, phénylalkyle en C₇-C₁₂ ou phénylalcoxy en C₇-C₁₂, les groupes b étant éventuellement substitués par un ou plusieurs restes du groupe constitué par les restes halogéno, alkyle en C₁-C₄, halogénoalkyle en C₁-C₃ et cyano.

4. Composés de formule (I) selon l'une ou plusieurs des revendications 1 à 3, choisi dans les groupes (Ia) à (Ie) 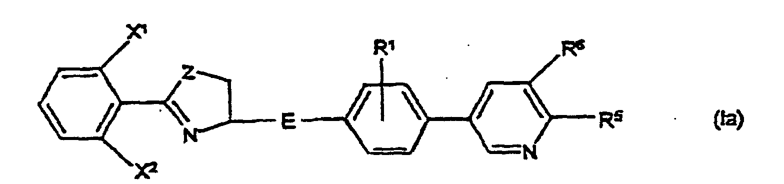 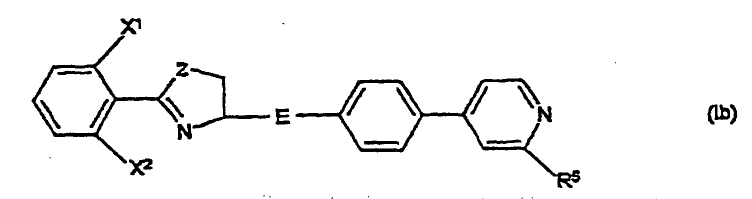 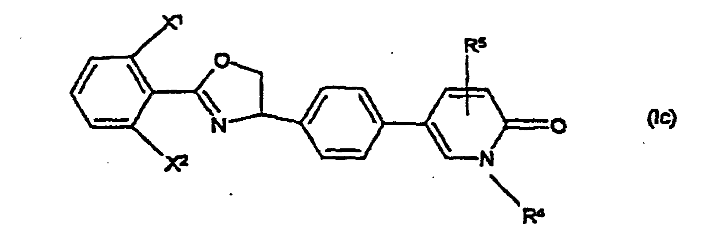 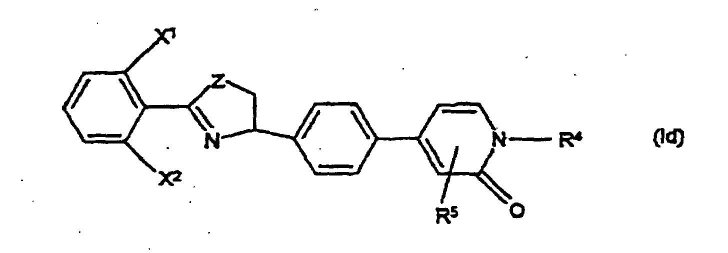 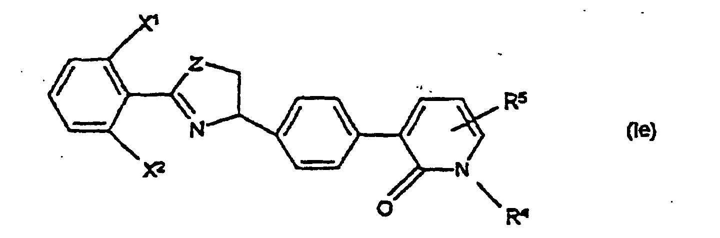 où les symboles ont la signification donnée pour la formule (I) dans les revendications 1 à 3.

5. Composés de formule (I) selon la revendication 4, choisis dans le groupe de (Ia1) à (Ia4): 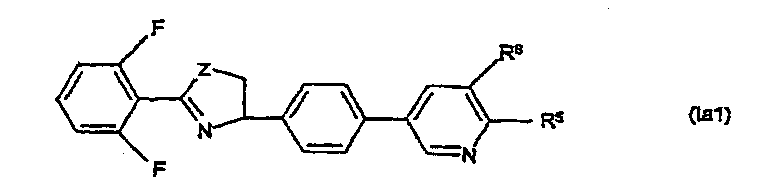 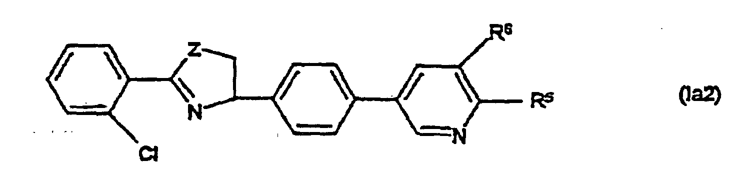 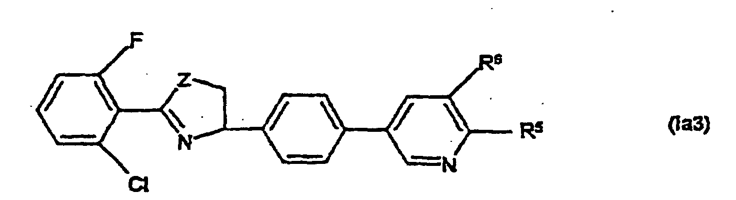 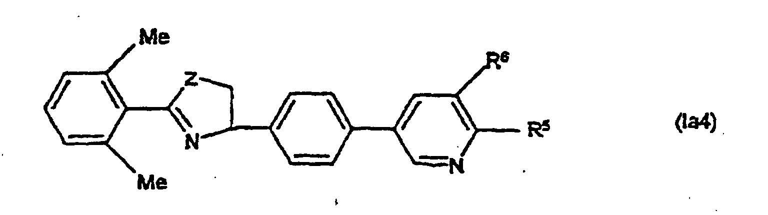 où les symboles ont la signification donnée pour la formule (I) dans les revendications 1 à 3.

6. Procédé de préparation de composés de formule (I) selon l'une ou plusieurs des revendications 1 à 5, **caractérisé en ce que** l'on fait réagir un composé halogéné ou un composé de perfluoroalkylsulfonate de formule (II) 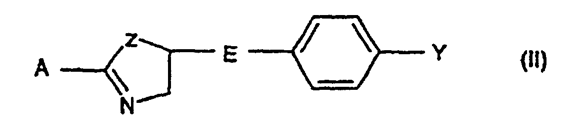 dans laquelle
Y représente Cl, Br, I ou un groupe perfluoroalkylsulfonate, et
A, Z et E ont la signification donnée pour la formule (I),
dans des conditions de catalyse avec du palladium, avec un composé organométallique de formule (III)
G-M (III)
dans laquelle
M représente un groupe partant contenant B, Sn ou Zn et
G a la signification donnée pour la formule (I) dans la revendication 1.

7. Composition phytosanitaire contenant au moins un composé selon l'une des revendications 1 à 5 et au moins un agent de formulation.

8. Composition insecticide, acaricide ou nématicide selon la revendication 7, contenant une quantité active d'au moins un composé selon l'une des revendications 1 à 5 avec les additifs ou substances auxiliaires habituels pour cette application.

9. Composition phytosanitaire, contenant une quantité active comme insecticide, acaricide ou nématicide d'au moins un composé selon l'une des revendications 1 à 5 et au moins une autre substance active, avec les additifs ou substances auxiliaires habituels pour cette application.

10. Composition à appliquer dans la protection du bois et comme conservateur dans des compositions d'étanchéité, dans des peintures, dans des réfrigérants lubrifiants pour le traitement des métaux ou dans des huiles à forer ou des huiles de coupe, contenant une quantité active d'au moins un composé selon l'une des revendications 1 à 5 avec les additifs et les substances auxiliaires habituels pour ces applications.

11. Composé selon l'une des revendications l'à 5 ou composition selon la revendication 7, 8 ou 9, à appliquer comme médicament vétérinaire.

12. Procédé de préparation d'une composition selon l'une des revendications 7 à 10, **caractérisé en ce que** l'on mélange la substance active et les autres additifs et on les met sous une forme d'application appropriée.

13. Utilisation d'un composé selon l'une des revendications 1 à 5 ou d'une composition selon l'une des revendications 7 et 10 comme agent de protection du bois ou comme conservateur dans des compositions d'étanchéité, dans des peintures, dans des réfrigérants lubrifiants pour le traitement des métaux ou dans des huiles à forer ou des huiles de coupe.

14. Utilisation de composés selon l'une des revendications 1 à 5 ou d'une composition selon l'une des revendications 7, 8 ou 9, pour la lutte contre les insectes nuisibles, les acariens, les mollusques et les nématodes.

15. Procédé de lutte contre les insectes nuisibles, les acariens, les mollusques et les nématodes, dans lequel on applique sur ceux-ci ou sur les plantes, les surfaces ou les substrats qu'ils infestent une quantité active d'un composé selon l'une des revendications 1 à 5 ou d'une composition selon l'une des revendications 7, 8 ou 9.

16. Semences enrobées par une quantité efficace d'un composé selon l'une des revendications 1 à 5 ou d'une composition selon l'une des revendications 7, 8 ou 9.
